# EUROPEAN PATENT APPLICATION

(11) **EP 4 722 256 A1**
(43) Date of publication of application: **08.04.2026**
(21) Application number: 24814411.5
(22) Date of filing: 28.05.2024
(51) Int. Cl.: C07K 19/00, C12N 15/62, C12N 15/867, C12N 5/10, A61K 45/06, A61K 39/00

(54) **DESIGN AND PREPARATION METHOD AND USE OF NOVEL AND MINIATURIZED CAR MOLECULE**

(30) Priority: 31.05.2023 CN 202310631350
(71) Applicant: CR Therapeutics Co., Ltd., (Sichuan) Free Trade Pilot Zone 610213 (CN)
(72) Inventor: YANG, Hanshuo, Chengdu, Sichuan 610041 (CN); LUO, Hui, Chengdu, Sichuan 610041 (CN)
(74) Representative: Novagraaf Group
(86) International application number: PCT/CN2024/095655
(87) International publication number: WO 2024/245214

(57) **Abstract**

The present invention relates to a novel miniaturized CAR molecule design, a preparation method and an application therefor. The novel miniaturized chimeric antigen receptor comprising an extracellular segment, a transmembrane region and an intracellular segment. The extracellular segment comprises an antigen-binding region and a hinge region; the intracellular segment comprises a portion of CD3ζ in tandem with a co-stimulatory molecule. The chimeric antigen receptor has a length of equal to or less than 300 amino acids; the antigen-binding region of the extracellular segment has a length of equal to or less than 150 amino acids; the hinge region and the transmembrane region have a combined length of equal to or less than 70 amino acids; and the intracellular segment has a length of equal to or less than 100 amino acids.

## Description

### REFERENCE TO RELATED APPLICATIONS

This application claims the priority to Chinese patent application No. 202310631350.7, titled "A Novel CAR-T Cell Targeting HER2-Positive Tumors, and Preparation Method and Application Thereof", filed on 31 May 2023, the contents of which are incorporated herein by reference.

### TECHNICAL FIELD

The present disclosure relates generally to the field of molecular cell biology, and specifically relates to a novel miniaturized CAR molecule design, a preparation method and an application thereof.

### BACKGROUND

CAR-T cell therapy (Chimeric Antigen Receptor T-Cell Immunotherapy) is a novel precision-targeted therapy for treating tumors. Research has shown that CAR-T cells can accurately, rapidly, efficiently and specifically recognize and kill tumor cells. In recent years, through continuous optimization and improvement, CAR-T cell therapy has achieved good results in assisting tumor treatment. It represents a highly promising research direction in tumor immunotherapy and has the potential to cure certain cancers. However, the cytotoxic effect of CAR-T cell therapy varies among different tumor types and remains controversial, especially in solid tumors, which presents a challenge that urgently requires technical breakthroughs.

The concept and design of CAR molecules have undergone multiple generations of development. The first-generation CARs were developed in the late 1990s and contained a full-length CD3ζ or FcRy signaling domain. Due to poor durability and persistence in vitro, these CAR-T cells exhibited limited or no clinical efficacy. Subsequently, second-generation CARs were developed, in which a single co-stimulatory domain was linked to the CD3ζ intracellular signaling domain. Among existing FDA-approved CAR-T products, the most commonly used co-stimulatory domains are CD28 and 4-1BB (CD137). Although second-generation CAR-T cells have demonstrated good clinical efficacy in hematologic malignancies, including various blood cancers, their effectiveness in solid tumors, such as glioblastoma, advanced sarcoma, liver metastases and ovarian cancer, remains poor. To achieve better therapeutic outcomes, researchers have further designed third-generation CARs by combining two co-stimulatory domains in tandem with the CD3ζ intracellular signaling domain to deliver stronger T-cell stimulatory signals. However, third-generation CAR-T cells have not shown significant improvements in therapeutic effects and instead were found to cause substantial toxicity, resulting in decreased research interest in this design. Fourth-generation CARs incorporate additional functional elements into the traditional CAR structure, such as cytokine expression modules, to enhance immune activation and anti-tumor activity. However, the fourth-generation CARs remain in preliminary research stages, and their therapeutic efficacy requires further validation.

More and more research has shown that even minor changes in any part of the CAR structure may influence the anti-tumor activity of CAR-T cells. In particular, differences in the scFv, hinge region, transmembrane region, co-stimulatory domain or ITAMs signaling domains may affect the effectiveness or safety of CAR-T cells. This is because T cells require antigen stimulation to become activated and expand; however, excessive activation or excessively strong affinity toward a target may instead lead to T-cell exhaustion and reduced cytotoxic activity, thereby impairing the durability of CAR-T cell function.

In summary, the present invention attempts to provide a structurally improved novel miniaturized CAR design and corresponding CAR-T cells to alleviate at least one of the aforementioned problems.

### SUMMARY

In view of the foregoing, an aspect of the present invention is to provide a novel miniaturized CAR design, a preparation method and an application therefor, and the specific technical solutions are as follows.

A novel miniaturized chimeric antigen receptor, comprising an extracellular segment, a transmembrane region and an intracellular segment, wherein the extracellular segment comprises an antigen-binding region and a hinge region; the intracellular segment comprises a portion of CD3ζ in tandem with a co-stimulatory molecule; the chimeric antigen receptor has a length of equal to or less than 300 amino acids; the antigen-binding region of the extracellular segment has a length of equal to or less than 150 amino acids; the hinge region and the transmembrane region have a combined length of equal to or less than 70 amino acids; the intracellular segment has a length of equal to or less than 100 amino acids.

In some embodiments, the chimeric antigen receptor has a length of 280 to 300 amino acids.

In some embodiments, the portion of CD3ζ is a CD3ζ ITAM domain.

In some embodiments, the CD3ζ ITAM domain comprises a CD3ζ ITAM1 domain, a CD3ζ ITAM2 domain or a CD3ζ ITAM3 domain.

In some embodiments, the portion of CD3ζ is a CD3ζ ITAM1 domain, and the nucleotide sequence of the CD3ζ ITAM1 domain is shown as SEQ ID NO:2.

In some embodiments, the antigen-binding region is an antibody targeting an antigen of a solid tumor or an antigen of a hematologic tumor cell.

In some embodiments, the antibody comprises a single-domain antibody or a nanobody.

In some embodiments, the co-stimulatory molecule is a single co-stimulatory molecule.

In some embodiments, the hinge region comprises a CD8 hinge region or a CD28 hinge region.

Further, the transmembrane region comprises a CD8 transmembrane region, a CD28 transmembrane region or a CD3 transmembrane region.

In some embodiments, the hinge region is connected to the transmembrane region, the connections comprising a CD8 hinge region connected to a CD8 transmembrane region (8H+8TM), a CD28 hinge region connected to a CD28 transmembrane region (28H+28TM), a CD28 hinge region connected to a CD3 transmembrane region (28H+3TM), or a CD8 hinge region connected to a CD3 transmembrane region (8H+3TM).

A CAR-T cell expressing the above chimeric antigen receptor.

Use of the CAR-T cell in the manufacture of a medicament for the treatment of a solid tumor or a hematologic tumor.

Use of the CAR-T cell in the manufacture of a medicament for the treatment of an autoimmune disease.

In some embodiments, the solid tumor comprises one or more of ovarian cancer, breast cancer, gastric cancer, colorectal cancer, lymphoma and multiple myeloma with a target being positively expressed; the hematologic tumor comprises leukemia.

In some embodiments, a portion of a CD3ζ ITAM domain of the CAR-T cell is used for achieving appropriate activation of downstream signaling of a T cell.

### Technical Advantages of the Present Invention

The CAR structure provided in the present invention is a novel miniaturized second-generation CAR structure. This novel miniaturized second-generation CAR structure is short in length (both the extracellular segment and the intracellular segment are relatively short); it exhibits a high expression level, which may be increased by approximately two-fold compared with an ordinary CAR; it can effectively activate CAR-T cells to kill tumor cells, such that, at a low effector-to-target ratio (effector-to-target ratio of 1:1), the killing activity at 24 hours may be increased by up to approximately seven-fold; and the onset time of the killing activity is short, such that, at an effector-to-target ratio of 5:1, a killing effect may be observed as early as approximately six hours. In addition, the use of hinge regions or transmembrane regions derived from different molecules does not affect the killing activity of the novel miniaturized second-generation CAR. These results indicate that the novel miniaturized second-generation CAR provided in the present invention can significantly improve treatment effectiveness.

Specifically, a protein sequence of the CAR structure provided in the present invention has a length of less than 300 amino acids, and a portion of a CD3ζ signalling domain is linked only to a single intracellular co-stimulatory molecule (for example, 4-1BB). The present invention demonstrates that such a miniaturized CAR can exhibit good tumor-killing effects in various tumors, including solid tumors and hematologic tumors. The CD3ζ signalling domain comprises CD3ζ ITAM1, ITAM2 and ITAM3. Currently, there have been no reports that a CAR constructed using only a single CD3ζ ITAM domain, or the corresponding CAR-T cells prepared therefrom, can exhibit good killing activity against solid tumors; therefore, the present invention represents a technical advancement at least to some extent. The present invention verifies through experiments that CAR-T cells constructed using only a single CD3ζ ITAM1 can exhibit good tumor-killing effects; however, this example is provided for illustration only and is not intended to define the scope of the present invention.

Finally, the CAR-T cells prepared in the present invention retain only a single CD3ζ ITAM domain. Experimental results demonstrate that this single CD3ζ ITAM domain is sufficient to activate downstream signalling of a T cell, enabling CAR-T cells to exert effective tumor-killing activity. In addition, the use of only a portion of CD3ζ can prevent excessive activation of downstream T-cell signalling, thereby avoiding damage to CAR-T cells and enabling better persistence of killing activity. Accordingly, the CAR-T cells prepared in the present invention not only exhibit effective killing capacity, but also demonstrate improved durability of tumor-killing function.

### BRIEF DESCRIPTION OF THE DRAWINGS

To more clearly illustrate the technical solutions of the embodiments of the present invention or of the prior art, the drawings required for describing the embodiments or the prior art are briefly introduced below. It is apparent that the drawings described below represent only some embodiments of the present invention, and for a person of ordinary skill in the art, other drawings may also be obtained based on these drawings without requiring inventive effort.
[Fig. 1]: A schematic diagram of the novel miniaturized CAR structure synthesized in the present invention.
[Fig. 2]: A result diagram showing the positive expression rate of the HER2 CAR synthesized in one embodiment of the present invention (APC is the fluorophore; H represents height; the horizontal axis represents fluorescence intensity).
[Fig. 3]: A diagram showing the in vitro killing effect (LDH) of the HER2 CAR-T cells in one embodiment of the present invention.
[Fig. 4]: A diagram showing the killing effect at 24 hours for SKOV3-mCherry tumor cells and HER2 CAR-T cells at an effector-to-target ratio of 1:1 in one embodiment of the present invention (magnification: 100×).
[Fig. 5]: A curve chart showing the continuous killing of SKOV3-mCherry tumor cells by HER2 CAR-T cells at an effector-to-target ratio of 1:1 in one embodiment of the present invention.
[Fig. 6]: A diagram showing the killing effect at 24 hours for SKOV3-mCherry tumor cells and HER2 CAR-T cells at an effector-to-target ratio of 1:2.5 in one embodiment of the present invention (magnification: 100×).
[Fig. 7]: A curve chart showing the continuous killing of SKOV3-mCherry tumor cells by HER2 CAR-T cells at an effector-to-target ratio of 1:2.5 in one embodiment of the present invention.
[Fig. 8]: A diagram showing the killing effect at 24 hours for SKOV3-mCherry tumor cells and HER2 CAR-T cells at an effector-to-target ratio of 1:5 in one embodiment of the present invention (magnification: 100×).
[Fig. 9]: A curve chart showing the continuous killing of SKOV3-mCherry tumor cells by HER2 CAR-T cells at an effector-to-target ratio of 1:5 in one embodiment of the present invention.
[Fig. 10]: A result diagram showing the positive expression rate of the CLDN 18.2 CAR synthesized in one embodiment of the present invention (PE is the fluorophore; A represents area; the horizontal axis represents fluorescence intensity).
[Fig. 11]: A diagram showing the in vitro killing effect (LDH) of CLDN 18.2 CAR-T cells in one embodiment of the present invention.
[Fig. 12]: A diagram showing the killing effect at 24 hours for SKOV3-mCherry-CLDN 18.2 tumor cells and CLDN 18.2 CAR-T cells at an effector-to-target ratio of 1:1 in one embodiment of the present invention (magnification: 100×).
[Fig. 13]: A curve chart showing the continuous killing of SKOV3-mCherry-CLDN 18.2 tumor cells by CLDN 18.2 CAR-T cells at an effector-to-target ratio of 1:1 in one embodiment of the present invention.
[Fig. 14]: A diagram showing the killing effect at 24 hours for SKOV3-mCherry-CLDN 18.2 tumor cells and CLDN 18.2 CAR-T cells at an effector-to-target ratio of 1:2.5 in one embodiment of the present invention (magnification: 100×).
[Fig. 15]: A curve chart showing the continuous killing of SKOV3-mCherry-CLDN 18.2 tumor cells by CLDN 18.2 CAR-T cells at an effector-to-target ratio of 1:2.5 in one embodiment of the present invention.
[Fig. 16]: A diagram showing the killing effect at 24 hours for SKOV3-mCherry-CLDN 18.2 tumor cells and CLDN 18.2 CAR-T cells at an effector-to-target ratio of 1:5 in one embodiment of the present invention (magnification: 100×).
[Fig. 17]: A curve chart showing the continuous killing of SKOV3-mCherry-CLDN 18.2 tumor cells by CLDN 18.2 CAR-T cells at an effector-to-target ratio of 1:5 in one embodiment of the present invention.
[Fig. 18]: A result diagram showing the positive expression rate of the BCMA CAR synthesized in one embodiment of the present invention (APC is the fluorophore; A represents area; the horizontal axis represents fluorescence intensity).
[Fig. 19]: A diagram showing the in vitro killing effect (LDH) of BCMA CAR-T cells in one embodiment of the present invention.
[Fig. 20]: A diagram showing the killing effect at 24 hours for MM1S-mCherry tumor cells and BCMA CAR-T cells at an effector-to-target ratio of 1:2.5 in one embodiment of the present invention (magnification: 100×).
[Fig. 21]: A curve chart showing the continuous killing of MM1S-mCherry tumor cells by BCMA CAR-T cells at an effector-to-target ratio of 1:2.5 in one embodiment of the present invention.
[Fig. 22]: A diagram showing the killing effect at 24 hours for MM1S-mCherry tumor cells and BCMA CAR-T cells at an effector-to-target ratio of 1:5 in one embodiment of the present invention (magnification: 100×).
[Fig. 23]: A curve chart showing the continuous killing of MM1S-mCherry tumor cells by BCMA CAR-T cells at an effector-to-target ratio of 1:5 in one embodiment of the present invention.
[Fig. 24]: A diagram showing the killing effect at 24 hours for MM1S-mCherry tumor cells and BCMA CAR-T cells at an effector-to-target ratio of 1:10 in one embodiment of the present invention (magnification: 100×).
[Fig. 25]: A curve chart showing the continuous killing of MM1S-mCherry tumor cells by BCMA CAR-T cells at an effector-to-target ratio of 1:10 in one embodiment of the present invention.
[Fig. 26]: A diagram showing the killing effect at 24 hours for SKOV3-mCherry tumor cells and HER2 CAR-T cells (with different transmembrane regions and hinge regions) at effector-to-target ratios of 1:2.5 and 1:5 in one embodiment of the present invention (magnification: 100×).
[Fig. 27]: A result diagram showing the positive expression rate of HER2 CARs with different structural designs synthesized in one embodiment of the present invention (APC is the fluorophore; H represents height; the horizontal axis represents fluorescence intensity).
[Fig. 28]: A diagram showing the in vitro killing effect (LDH) of HER2 CAR-T cells with different structural designs in one embodiment of the present invention.
[Fig. 29]: A diagram showing the killing effect at 24 hours for SKOV3-mCherry tumor cells and HER2 CAR-T cells with different structural designs at an effector-to-target ratio of 1:1 in one embodiment of the present invention (magnification: 100×).
[Fig. 30]: A curve chart showing the continuous killing of SKOV3-mCherry tumor cells by HER2 CAR-T cells with different structural designs at an effector-to-target ratio of 1:1 in one embodiment of the present invention.
[Fig. 31]: A diagram showing the killing effect at 24 hours for SKOV3-mCherry tumor cells and HER2 CAR-T cells with different structural designs at an effector-to-target ratio of 1:2.5 in one embodiment of the present invention (magnification: 100×).
[Fig. 32]: A curve chart showing the continuous killing of SKOV3-mCherry tumor cells by HER2 CAR-T cells with different structural designs at an effector-to-target ratio of 1:2.5 in one embodiment of the present invention.
[Fig. 33]: A diagram showing the killing effect at 24 hours for SKOV3-mCherry tumor cells and HER2 CAR-T cells with different structural designs at an effector-to-target ratio of 1:5 in one embodiment of the present invention (magnification: 100×).
[Fig. 34]: A curve chart showing the continuous killing of SKOV3-mCherry tumor cells by HER2 CAR-T cells with different structural designs at an effector-to-target ratio of 1:5 in one embodiment of the present invention.

### DETAILED DESCRIPTION

To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

As used herein, the term "and/or" refers to any and all combinations of one or more of the associated listed items.

As used herein, "a plurality of" means two or more, including but not limited to two, three, four, five, and so on.

It should be noted that the term "include", "comprise" or any variant thereof is intended to encompass nonexclusive inclusion so that a process, method, article or device including a series of elements includes not only those elements but also other elements which have been not listed definitely or an element(s) inherent to the process, method, article or device. Moreover, the expression "comprising a(n) . . . " in which an element is defined will not preclude presence of an additional identical element(s) in a process, method, article or device comprising the defined element(s) unless further defined.

As used in the present specification, the term "about" typically represents ±5% of the stated value, more typically ±4%, more typically ±3%, more typically ±2%, even more typically ±1%, and in some cases even more typically ±0.5%.

In the present specification, certain embodiments may be disclosed in the form of being "within a range." It should be understood that such descriptions of being "within a range" are provided merely for convenience and brevity, and should not be construed as a rigid limitation of the disclosed range. Accordingly, a disclosed range should be regarded as specifically disclosing all possible sub-ranges and individual values within that range. For example, a disclosure of a range of 1 to 6 should be considered as specifically disclosing sub-ranges such as 1 to 3, 1 to 4, 1 to 5, 2 to 4, 2 to 6, 3 to 6, etc., as well as individual values within the range, such as 1, 2, 3, 4, 5 and 6. These rules apply regardless of the breadth of the range.

The term "appropriate activation" as used in the present invention refers to effectively activating downstream signalling of a T cell such that a CAR-T cell can exert effective killing against tumor cells, while at the same time avoiding excessive activation of downstream T-cell signalling that may otherwise cause damage to the CAR-T cell itself.

### EMBODIMENT 1

This embodiment provides an example of a preparation method for a CAR-T cell.

### 1. Construction of the CAR expression vector

(1) The CAR structure comprises an extracellular HER2 nanobody, a CD8 hinge region, a CD8 transmembrane region, an intracellular co-stimulatory molecule (4-1BB) and a portion of a CD3ζ signaling domain (CD3ζ ITAM1) / a full-length CD3ζ signaling domain (control). The structural fragments were prepared through gene synthesis.
   It is to be understood that the extracellular nanobody may be selected from various nanobodies targeting antigens of solid tumors or hematologic tumors. The nanobodies illustrated in the following embodiments are provided only as examples and should not be construed as exclusive. In this embodiment, a nanobody targeting HER2 is used as an example.
(2) The Pwpxld vector and the CAR structural fragment were each digested with the restriction endonucleases EcoRI and BamHI. After ligation using T4 ligase, transformation and screening were performed. Positive clones obtained were subjected to sequencing analysis, ultimately yielding the Her2 VH-BBZ(short)-Pwpxld expression vector and the Her2 VH-BBZ(full)-Pwpxld control vector (containing full-length CD3ζ).

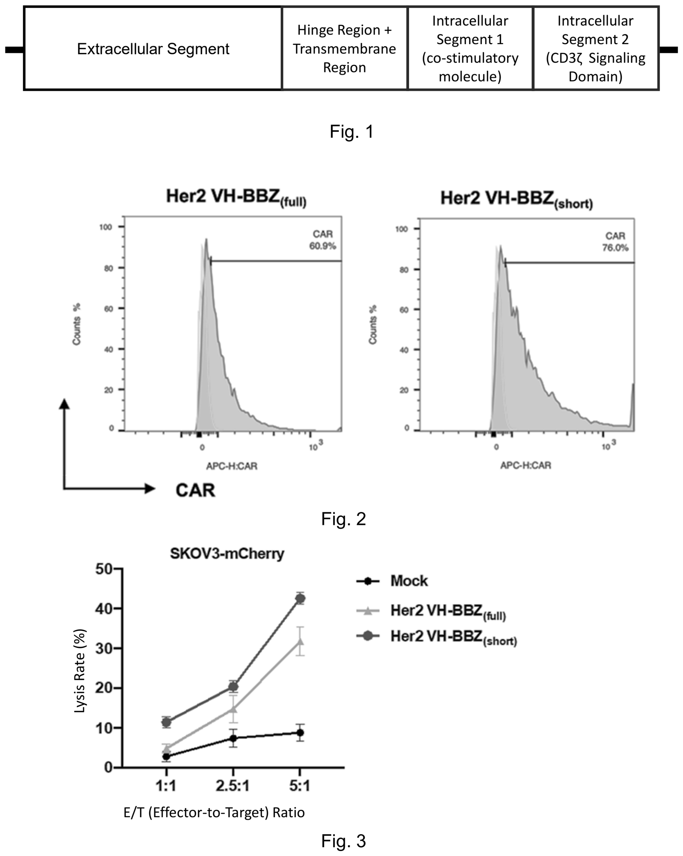

The CAR structure of the present invention is shown in Fig. 1.

The sequences of the respective fragments synthesized/amplified in this embodiment are shown in Table 1.

**Table 1**

| NO. | Sequence | |
|---|---|---|
| 1 | Her2 CAR | ATGGCCTTACCAGTGACCGCCTTGCTCCTGCCGCTGGCCTTGCTGCTCCAC GCCGCCAGGCCGGAGGTGCAGCTGGTGGAGAGCGGGGGGGGACTGGTGC AGGCAGGAGGAAGCCTGAGACTGAGCTGTGCCGCAAGCGGGATTACATTTA GTATCAACACAATGGGATGGTACAGGCAGGCTCCTGGCAAGCAGAGAGAGC TGGTGGCTCTGATTTCAAGCATCGGAGACACCTACTACGCCGACTCTGTGAA AGGACGGTTCACCATCAGCAGAGACAACGCAAAGAACACCGTCTACCTGCA GATGAACAGCCTGAAACCTGAAGACACAGCAGTGTACTATTGCAAAAGGTTC CGAACCGCTGCTCAGGGCACTGACTACTGGGGCCAGGGCACCCAGGTGAC CGTGTCCAGCACCACGACGCCAGCGCCGCGACCACCAACACCGGCGCCCA CCATCGCGTCGCAGCCCCTGTCCCTGCGCCCAGAGGCGTGCCGGCCAGCG GCGGGGGGCGCAGTGCACACGAGGGGGCTGGACTTCGCCTGTGATATCTA CATCTGGGCGCCCCTGGCCGGGACTTGTGGGGTCCTTCTCCTGTCACTGGT TATCACCAAACGGGGCAGAAAGAAACTCCTGTATATATTCAAACAACCATTTA TGAGACCAGTACAAACTACTCAAGAGGAAGATGGCTGTAGCTGCCGATTTCC AGAAGAAGAAGAAGGAGGATGTGAACTGGCCCCCGCGTACCAGCAGGGCC AGAACCAGCTCTATAACGAGCTCAATCTAGGACGAAGAGAGGAGTACGATG TTTTGCACATGCAGGCCCTGCCCCCTCGCTGA (281 amino acids) |
| 2 | CD3ζ ITAM1 | GCCCCCGCGTACCAGCAGGGCCAGAACCAGCTCTATAACGAGCTCAATCTA GGACGAAGAGAGGAGTACGATGTTTTGCACATGCAGGCCCTGCCCCCTCGC (34 amino acids) |
| 3 | HER2 Nano body | GAGGTGCAGCTGGTGGAGAGCGGGGGGGGACTGGTGCAGGCAGGAGGAA GCCTGAGACTGAGCTGTGCCGCAAGCGGGATTACATTTAGTATCAACACAAT GGGATGGTACAGGCAGGCTCCTGGCAAGCAGAGAGAGCTGGTGGCTCTGA TTTCAAGCATCGGAGACACCTACTACGCCGACTCTGTGAAAGGACGGTTCA CCATCAGCAGAGACAACGCAAAGAACACCGTCTACCTGCAGATGAACAGCC TGAAACCTGAAGACACAGCAGTGTACTATTGCAAAAGGTTCCGAACCGCTGC TCAGGGCACTGACTACTGGGGCCAGGGCACCCAGGTGACCGTGTCCAGC (118 amino acids) |
| 4 | 3D8H+ CD8T M | ACCACGACGCCAGCGCCGCGACCACCAACACCGGCGCCCACCATCGCGTC GCAGCCCCTGTCCCTGCGCCCAGAGGCGTGCCGGCCAGCGGCGGGGGGC GCAGTGCACACGAGGGGGCTGGACTTCGCCTGTGATATCTACATCTGGGCG CCCCTGGCCGGGACTTGTGGGGTCCTTCTCCTGTCACTGGTTATCACC (66 amino acids) |

### 2. Lentiviral packaging

(1) Thaw and culture 293T cells; when the confluency reaches approximately 80%, proceed with viral packaging.
(2) Add chloroquine solution to the culture medium (DMEM + 10% FBS) to a final concentration of 100 µmol/L.
(3) The viral packaging system (per 10-cm culture dish) comprises: 50 µL CaCl₂, 20 µg of the constructed core plasmid, 10 µg Pspax2, 5 µg PMD2.0G, 500 µL nuclease-free water, and 500 µL 2× BBS.
(4) After approximately 10 hours, replace with fresh pre-warmed culture medium (DMEM + 10% FBS).
(5) Collect the viral supernatants at 48 and 72 hours of culture; filter using a 0.22-µm membrane to remove impurities.
(6) Ultracentrifuge, resuspend the concentrated virus in DPBS at 1/400 of the original volume of the viral supernatant, aliquot according to subsequent usage requirements, and store at -80 °C for later use.

### 3. Isolation of peripheral blood cells

(1) Pre-coat T-cell culture flasks (T25) one day in advance and incubate overnight at 4 °C. The T-cell culture medium contains anti-human CD3 and CD28 monoclonal antibodies: 3 mL DPBS, 4 µg/mL CD3, and 2 µg/mL CD28.
(2) Collect an appropriate amount of freshly drawn blood from a healthy volunteer.
(3) Add 5 mL Ficoll to a 15-mL BD tube, and slowly layer 5 mL of fresh blood on top.
(4) Centrifuge at room temperature at 1000 g (centrifuge acceleration: up 1, down 1) for 30 minutes.
(5) After centrifugation, carefully aspirate the middle (white) layer containing peripheral blood lymphocytes.
(6) Wash with three volumes of pre-warmed DPBS, centrifuge at 300 g for 10 minutes.
(7) Optionally, add 5 mL red blood cell lysis buffer and lyse at room temperature for 5 minutes, then add an equal volume of pre-warmed DPBS.
(8) Count the cells, then centrifuge at room temperature at 100 g for 10 minutes.
(9) Resuspend the cells in an appropriate amount of T-cell culture medium (X-Vivo, 5% AB serum, 400 IU IL-2) at 2 × 10⁶ cells/mL.
(10) Remove the antibody incubation solution from the T25 flask, add the T-cell suspension, and culture normally at 37 °C for approximately 48 hours.

### 4. Preparation of CAR-T cells

(1) Pre-coat the T-cell culture plate one day in advance and incubate overnight at 4 °C. A 24-well plate is used, and each well is supplemented with 0.5 mL DPBS and 10 µL RetroNectin (1 mg/mL).
(2) Remove the RetroNectin solution.
(3) Add 1 mL 2% BSA solution to each well and block at 37 °C for 30 minutes.
(4) Remove the blocking solution and wash once with DPBS.
(5) To each well (non-adherent plate), add 200 µL of 400× virus and 300 µL X-Vivo complete medium, then centrifuge at 32 °C, 1000 g (centrifuge acceleration: up 2, down 2) for 2 hours.
(6) Add 2-2.5 × 10⁶ T cells that have been activated for approximately 48 hours into each well, bring the total volume to 1 mL per well with X-Vivo complete medium, and centrifuge at 32 °C, 300 g (centrifuge acceleration: up 2, down 2) for 15 minutes.
   The miniaturized CAR-T cells synthesized in this embodiment are referred to as Her2 VH-BBZ(short), and the full-length control CAR-T cells are referred to as Her2 VH-BBZ(full).
(7) Culture normally at 37 °C for approximately 72 hours. Perform flow cytometry to detect the positive expression rate of CAR in the experimental and control groups, as shown in Fig. 2. The results show that the positive expression rate of Her2 VH-BBZ(short) is 76.0%, whereas that of Her2 VH-BBZ(full) is 60.9%.

### EMBODIMENT 2

This embodiment provides an in vitro killing assay for the CAR-T cells synthesized in Embodiment 1.
(1) 96-well plate killing assay: tumor cells were seeded at 1 × 10⁴ cells per well, and the CAR-T cells synthesized in Embodiment 1 were co-cultured with the tumor cells at effector-to-target ratios (effector cells : target cells = CAR-T : tumor cells) of 1:1, 2.5:1 and 5:1, respectively. Each effector-to-target ratio contained three replicate wells, and mock T cells (Mock) were used as a control.
   In this embodiment, the human ovarian cancer cell line SKOV3-mCherry was used. This cell line is a broadly representative tumor cell model.
(2) A live-cell imaging system was used to continuously monitor the in vitro killing activity of CAR-T cells in the experimental group and the control group.
(3) After approximately 24 hours of co-culture, the culture supernatant was collected to measure LDH for calculating the tumor lysis rate.

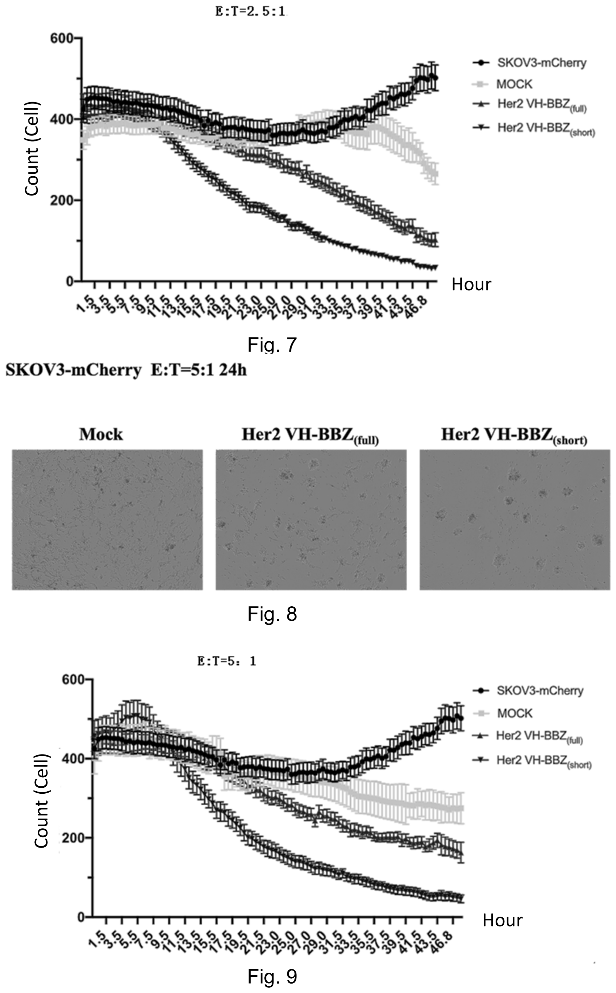

The experimental results are shown in Figs. 3-9.

Fig. 3 shows that both the miniaturized CAR-T cells synthesized in Embodiment 1 and the control CAR-T cells could exert effective killing against HER2-positive tumor cells. However, the Her2 VH-BBZ(short) CAR-T cells exhibited significantly stronger tumor-killing ability. At an effector-to-target ratio of E:T = 1:1, the killing effect of Her2 VH-BBZ(short) CAR-T cells was already obviously better than that of Her2 VH-BBZ(full) CAR-T cells; and at an effector-to-target ratio of E:T = 5:1, the killing effect showed an explosive trend, a phenomenon not observed in Her2 VH-BBZ(full) CAR-T cells at the same effector-to-target ratio.

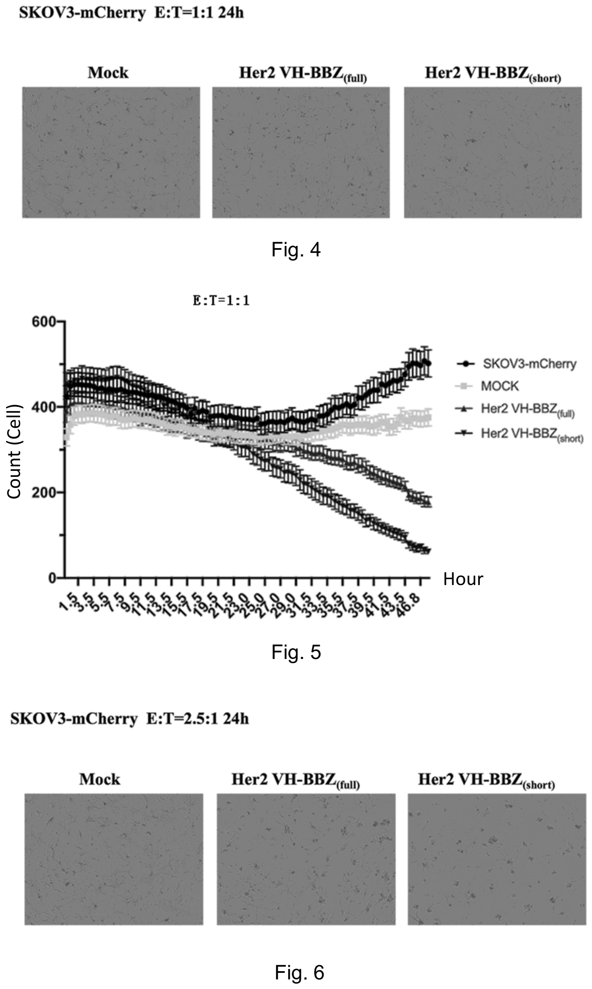

Specifically, Figs. 4-9 show the continuous killing effects under different effector-to-target ratios. Taking E:T = 1:1 as an example (E represents effector cells; T represents target tumor cells), the killing effects of Her2 VH-BBZ(short) CAR-T cells and Her2 VH-BBZ(full) CAR-T cells were not markedly different within the first 24 hours; however, after 24 hours, the killing activity of Her2 VH-BBZ(short) CAR-T cells gradually surpassed that of Her2 VH-BBZ(full) CAR-T cells, with the difference being most pronounced after 48 hours of continuous killing. These results indicate that a single CD3ζ ITAM domain (CD3ζ ITAM1) is sufficient to activate downstream signalling of a T cell, thereby promoting effective killing of tumor cells by CAR-T cells while avoiding excessive activation of downstream signalling that may damage CAR-T cells, ultimately contributing to better persistence of tumor-killing activity.

### EMBODIMENT 3

This embodiment provides another example of a preparation method for CAR-T cells.

### 1. Construction of the CAR expression vector

(1) The CAR structure comprises an extracellular CLDN18.2 nanobody, a CD8 hinge region, a CD8 transmembrane region, an intracellular co-stimulatory molecule (4-1BB) and a portion of a CD3ζ signalling domain (CD3ζ ITAM1) / a full-length CD3ζ signalling domain (control). The structural fragments were prepared through gene synthesis.
   In this embodiment, a nanobody targeting CLDN18.2 is used as an example.
(2) The Pwpxld vector and the CAR structural fragment were each digested with the restriction endonucleases EcoRI and BamHI. After ligation using T4 ligase, transformation and screening were performed. Positive clones obtained were subjected to sequencing analysis, ultimately yielding the CLDN18.2 VH-BBZ(short)-Pwpxld expression vector and the CLDN18.2 VH-BBZ(full)-Pwpxld control vector (containing full-length CD3ζ).

The sequences of the respective fragments synthesized/amplified in this embodiment are shown in Table 2.

**Table 2**

| NO. | Sequence | |
|---|---|---|
| 5 | CLDN1 8.2 CAR | ATGGCCTTACCAGTGACCGCCTTGCTCCTGCCGCTGGCCTTGCTGCTCCAC GCCGCCAGGCCGCAGGTCCAGCTGGTCGAGTCTGGAGGTGGCCTGGTCCA GCCAGGTGGCAGTCTGAGGCTGAGTTGCGCAGCCTCTGGAGTGGACATCT CTTCCGACGTGATGGCCTGGTATAGGCAGGCACCTGGTAAGGGACTGGAAT TTGTCTCTGGTCTGACTAGAGGTGGCAGCATCAACTACGCTGACAGCGTCA AGGGAAGGTTTACCATCAGCAGGGACAACTCCAAGAACACCTTGTACCTGC AGATGAACAGCCTGAGAGCCGAAGATACAGCCGTGTACTACTGCAACGCCG AAATCTACACCGGCACCTTCTACCCACGGTCTTACTGGGGCCAGGGCACAT TGGTAACCGTGTCAACCACGACGCCAGCGCCGCGACCACCAACACCGGCG CCCACCATCGCGTCGCAGCCCCTGTCCCTGCGCCCAGAGGCGTGCCGGCC AGCGGCGGGGGGCGCAGTGCACACGAGGGGGCTGGACTTCGCCTGTGATA TCTACATCTGGGCGCCCCTGGCCGGGACTTGTGGGGTCCTTCTCCTGTCAC TGGTTATCACCAAACGGGGCAGAAAGAAACTCCTGTATATATTCAAACAACC ATTTATGAGACCAGTACAAACTACTCAAGAGGAAGATGGCTGTAGCTGCCGA TTTCCAGAAGAAGAAGAAGGAGGATGTGAACTGGCCCCCGCGTACCAGCAG GGCCAGAACCAGCTCTATAACGAGCTCAATCTAGGACGAAGAGAGGAGTAC GATGTTTTGCACATGCAGGCCCTGCCCCCTCGCTGA (282 amino acids) |
| 6 | CLDN1 8.2 Nanob ody | CAGGTCCAGCTGGTCGAGTCTGGAGGTGGCCTGGTCCAGCCAGGTGGCAG TCTGAGGCTGAGTTGCGCAGCCTCTGGAGTGGACATCTCTTCCGACGTGAT GGCCTGGTATAGGCAGGCACCTGGTAAGGGACTGGAATTTGTCTCTGGTCT GACTAGAGGTGGCAGCATCAACTACGCTGACAGCGTCAAGGGAAGGTTTAC CATCAGCAGGGACAACTCCAAGAACACCTTGTACCTGCAGATGAACAGCCT GAGAGCCGAAGATACAGCCGTGTACTACTGCAACGCCGAAATCTACACCGG CACCTTCTACCCACGGTCTTACTGGGGCCAGGGCACATTGGTAACCGTGTC A (119 amino acids) |

### 2. Lentiviral packaging

(1) Thaw and culture 293T cells; when the confluency reaches approximately 80%, proceed with viral packaging.
(2) Add chloroquine solution to the culture medium (DMEM + 10% FBS) to a final concentration of 100 µmol/L.
(3) The viral packaging system (per 10-cm culture dish) comprises: 50 µL CaCl₂, 20 µg of the constructed core plasmid, 10 µg Pspax2, 5 µg PMD2.0G, 500 µL nuclease-free water, and 500 µL 2× BBS.
(4) After approximately 10 hours, replace with fresh pre-warmed culture medium (DMEM + 10% FBS).
(5) Collect the viral supernatants at 48 and 72 hours of culture; filter using a 0.22-µm membrane to remove impurities.
(6) Ultracentrifuge, resuspend the concentrated virus in DPBS at 1/400 of the original volume of the viral supernatant, aliquot according to subsequent usage requirements, and store at -80 °C for later use.

### 3. Isolation of peripheral blood cells

(1) Pre-coat T-cell culture flasks (T25) one day in advance and incubate overnight at 4 °C. The T-cell culture medium contains anti-human CD3 and CD28 monoclonal antibodies: 3 mL DPBS, 4 µg/mL CD3, and 2 µg/mL CD28.
(2) Collect an appropriate amount of freshly drawn blood from a healthy volunteer.
(3) Add 5 mL Ficoll to a 15-mL BD tube, and slowly layer 5 mL of fresh blood on top.
(4) Centrifuge at room temperature at 1000 g (centrifuge acceleration: up 1, down 1) for 30 minutes.
(5) After centrifugation, carefully aspirate the middle (white) layer containing peripheral blood lymphocytes.
(6) Wash with three volumes of pre-warmed DPBS, centrifuge at 300 g for 10 minutes.
(7) Optionally, add 5 mL red blood cell lysis buffer and lyse at room temperature for 5 minutes, then add an equal volume of pre-warmed DPBS.
(8) Count the cells, then centrifuge at room temperature at 100 g for 10 minutes.
(9) Resuspend the cells in an appropriate amount of T-cell culture medium (X-Vivo, 5% AB serum, 400 IU IL-2) at 2 × 10⁶ cells/mL.
(10) Remove the antibody incubation solution from the T25 flask, add the T-cell suspension, and culture normally at 37 °C for approximately 48 hours.

### 4. Preparation of CAR-T cells

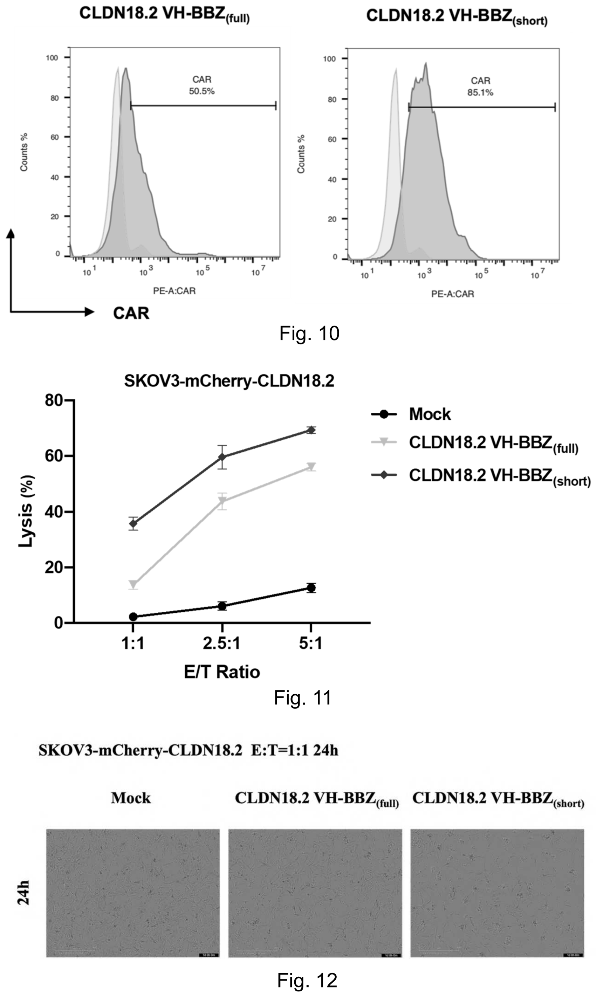

(1) Pre-coat the T-cell culture plate one day in advance and incubate overnight at 4 °C. A 24-well plate is used, and each well is supplemented with 0.5 mL DPBS and 10 µL RetroNectin (1 mg/mL).
(2) Remove the RetroNectin solution.
(3) Add 1 mL of 2% BSA solution to each well and block at 37 °C for 30 minutes.
(4) Remove the blocking solution and wash once with DPBS.
(5) To each well (non-adherent plate), add 200 µL of 400× virus and 300 µL X-Vivo complete medium, then centrifuge at 32 °C, 1000 g (centrifuge acceleration: up 2, down 2) for 2 hours.
(6) Add 2-2.5 × 10⁶ T cells that have been activated for approximately 48 hours into each well, bring the total volume to 1 mL per well with X-Vivo complete medium, and centrifuge at 32 °C, 300 g (centrifuge acceleration: up 2, down 2) for 15 minutes.
   The CAR-T cells synthesized in this embodiment are referred to as CLDN18.2 VH-BBZ(short), and the control CAR-T cells are referred to as CLDN18.2 VH-BBZ(full).
(7) Culture normally at 37 °C for approximately 72 hours. Perform flow cytometry to detect the positive expression rates of CAR in the experimental and control groups, as shown in Fig. 10. The results show that the positive expression rate of CLDN18.2 VH-BBZ(short) is 85.1%, whereas that of CLDN18.2 VH-BBZ(full) is 50.5%.

### EMBODIMENT 4

This embodiment provides an in vitro killing assay for the CAR-T cells synthesized in Embodiment 3.
(1) 96-well plate killing assay: Tumor cells were seeded at 1 × 10⁴ cells per well. The CAR-T cells synthesized in Embodiment 3 were co-cultured with the tumor cells at effector-to-target ratios of 1:1, 2.5:1 and 5:1, respectively. Each effector-to-target ratio contained three replicate wells, and mock T cells (Mock) were used as the control.
   In this embodiment, the SKOV3-mCherry-Claudin18.2 cell line was used, which is a tumor cell model overexpressing Claudin18.2 (CLDN18.2).
(2) A live-cell imaging system was used to continuously monitor the in vitro killing activity of CAR-T cells in the experimental group and the control group.
(3) After approximately 24 hours of co-culture, the culture supernatant was collected to measure LDH for calculating the tumor lysis rate.

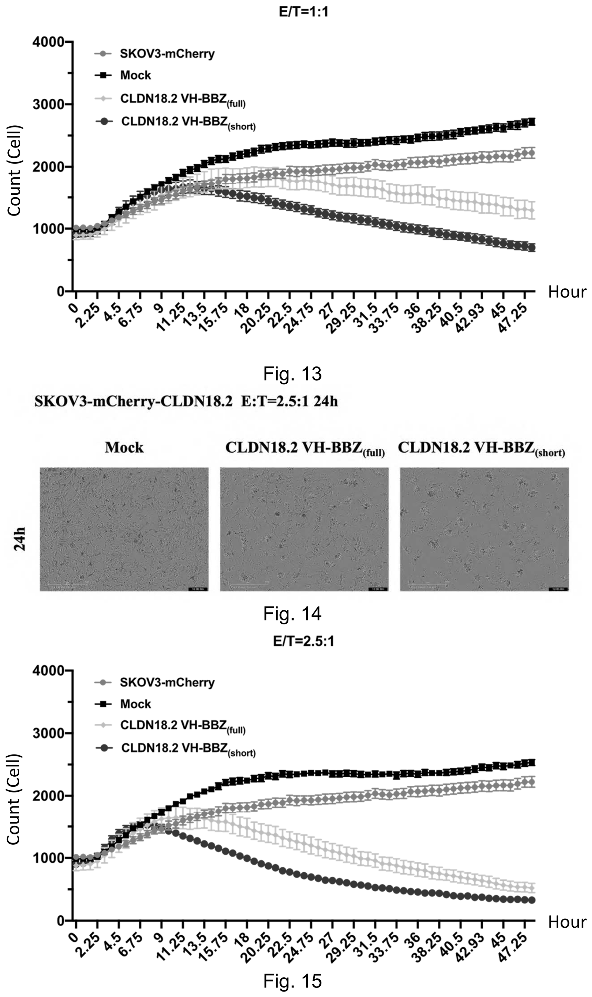

The experimental results are shown in Figs. 11-17.

Fig. 11 shows that both the miniaturized CAR-T cells synthesized in Embodiment 3 and the full-length control CAR-T cells could exert effective killing against CLDN18.2-positive tumor cells. However, the CLDN18.2 VH-BBZ(short) CAR-T cells exhibited significantly stronger tumor-killing ability. At an effector-to-target ratio of E:T = 1:1, the killing activity of CLDN18.2 VH-BBZ(short) CAR-T cells was already superior to that of CLDN18.2 VH-BBZ(full) CAR-T cells; moreover, this killing advantage was maintained at effector-to-target ratios of E:T = 2.5:1 and E:T = 5:1.

Specifically, Figs. 12-17 show the continuous killing effects under different effector-to-target ratios. Taking E:T = 1:1 as an example (E represents effector cells; T represents target tumor cells), after 24 hours of continuous killing, the killing effect of CLDN18.2 VH-BBZ(short) CAR-T cells was significantly better than that of CLDN18.2 VH-BBZ(full) CAR-T cells, and this killing advantage gradually increased over time. Compared with CLDN18.2 VH-BBZ(full) CAR-T cells, CLDN18.2 VH-BBZ(short) CAR-T cells exhibited an obviously superior killing effect after 10 hours of continuous killing at an effector-to-target ratio of E:T = 2.5:1, and after 8 hours of continuous killing at an effector-to-target ratio of E:T = 5:1. These results indicate that a single CD3ζ ITAM domain (CD3ζ ITAM1) is sufficient to activate downstream signalling of a T cell, thereby promoting effective killing of tumor cells by CAR-T cells while avoiding excessive activation of downstream signalling that may damage CAR-T cells, ultimately contributing to better persistence of tumor-killing activity.

### EMBODIMENT 5

This embodiment provides another example of a preparation method for CAR-T cells.

### 1. Construction of the CAR expression vector

(1) The CAR structure comprises an extracellular BCMA nanobody, a CD8 hinge region, a CD8 transmembrane region, an intracellular co-stimulatory molecule (4-1BB), and a portion of a CD3ζ signalling domain (CD3ζ ITAM1) / a full-length CD3ζ signalling domain (control). The structural fragments were prepared by gene synthesis.
   In this embodiment, a nanobody targeting BCMA is used as an example.
(2) The Pwpxld vector and the CAR structural fragment were each digested with the restriction endonucleases EcoRI and BamHI. After ligation using T4 ligase, transformation and screening were performed. Positive clones obtained were subjected to sequencing analysis, ultimately yielding the BCMA VH-BBZ(short)-Pwpxld expression vector and the BCMA VH-BBZ(full)-Pwpxld control vector (containing full-length CD3ζ).

The sequences of the respective fragments synthesized or amplified in this embodiment are shown in Table 3.

**Table 3**

| NO. | Sequence | |
|---|---|---|
| 7 | BCMA CAR | ATGGCCCTGCCTGTTACAGCTCTGCTGCTGCCTCTGGCTCTGCTTCTGCATG CCGCCAGACCTGAGGTGCAGCTGTTGGAGTCTGGGGGAGGCTTGGTACAG CCTGGGGGGTCCCTGAGACTCTCCTGTGCAGCCTCTGGATTCACCTTTAGC AGCTATGCCATGAGCTGGGTCCGCCAGGCTCCAGGGAAGGGGCTGGAGTG GGTCTCATCTATTAGTGGTAGTGGTGATTACATATACTACGCAGACTCCGTG AAGGGCCGGTTCACCATCTCCAGAGACATATCCAAGAACACGCTGTATCTG CAAATGAACAGCCTGAGAGCCGAGGACACGGCCGTATATTACTGTGCGAAA GAAGGTACGGGTGCCAACAGCAGCTTGGCAGACTACAGAGGCCAGGGCAC CCTGGTCACCGTCTCCTCATTCGTGCCCGTGTTCCTGCCTGCCAAGCCTAC CACGACGCCAGCGCCGCGACCACCAACACCGGCGCCCACCATCGCGTCGC AGCCCCTGTCCCTGCGCCCAGAGGCGTGCCGGCCAGCGGCGGGGGGCGC AGTGCACACGAGGGGGCTGGACTTCGCCTGTGATATCTACATCTGGGCGCC CCTGGCCGGGACTTGTGGGGTCCTTCTCCTGTCACTGGTTATCACCAAACG GGGCAGAAAGAAACTCCTGTATATATTCAAACAACCATTTATGAGACCAGTA CAAACTACTCAAGAGGAAGATGGCTGTAGCTGCCGATTTCCAGAAGAAGAA GAAGGAGGATGTGAACTGGCCCCCGCGTACCAGCAGGGCCAGAACCAGCT CTATAACGAGCTCAATCTAGGACGAAGAGAGGAGTACGATGTTTTGCACATG CAGGCCCTGCCCCCTCGCTGA (294 amino acids) |
| 8 | BCMA Nanob ody | GAGGTGCAGCTGTTGGAGTCTGGGGGAGGCTTGGTACAGCCTGGGGGGTC CCTGAGACTCTCCTGTGCAGCCTCTGGATTCACCTTTAGCAGCTATGCCATG AGCTGGGTCCGCCAGGCTCCAGGGAAGGGGCTGGAGTGGGTCTCATCTAT TAGTGGTAGTGGTGATTACATATACTACGCAGACTCCGTGAAGGGCCGGTT CACCATCTCCAGAGACATATCCAAGAACACGCTGTATCTGCAAATGAACAGC CTGAGAGCCGAGGACACGGCCGTATATTACTGTGCGAAAGAAGGTACGGGT GCCAACAGCAGCTTGGCAGACTACAGAGGCCAGGGCACCCTGGTCACCGT CTCCTCATTCGTGCCCGTGTTCCTGCCTGCCAAGCCT (131 amino acids) |

### 2. Lentiviral packaging

(1) Thaw and culture 293T cells; when the confluency reaches approximately 80%, proceed with viral packaging.
(2) Add chloroquine solution to the culture medium (DMEM + 10% FBS) to a final concentration of 100 µmol/L.
(3) The viral packaging system (per 10-cm culture dish) comprises: 50 µL CaCl₂, 20 µg of the constructed core plasmid, 10 µg Pspax2, 5 µg PMD2.0G, 500 µL nuclease-free water, and 500 µL 2× BBS.
(4) After approximately 10 hours, replace with fresh pre-warmed culture medium (DMEM + 10% FBS).
(5) Collect the viral supernatants at 48 and 72 hours of culture; filter using a 0.22-µm membrane to remove impurities.
(6) Ultracentrifuge, resuspend the concentrated virus in DPBS at 1/400 of the original volume of the viral supernatant, aliquot according to subsequent experimental requirements, and store at -80 °C for later use.

### 3. Isolation of peripheral blood cells

(1) Pre-coat T-cell culture flasks (T25) one day in advance and incubate overnight at 4 °C. The T-cell culture medium contains anti-human CD3 and CD28 monoclonal antibodies: 3 mL DPBS, 4 µg/mL CD3 and 2 µg/mL CD28.
(2) Collect an appropriate amount of freshly drawn blood from a healthy volunteer.
(3) Add 5 mL Ficoll to a 15-mL BD tube and gently layer 5 mL of fresh blood on top.
(4) Centrifuge at room temperature at 1000 g (centrifuge acceleration: up 1, down 1) for 30 minutes.
(5) After centrifugation, carefully aspirate the middle (white) layer containing peripheral blood lymphocytes.
(6) Wash with three volumes of pre-warmed DPBS and centrifuge at 300 g for 10 minutes.
(7) Optionally, add 5 mL red blood cell lysis buffer and lyse at room temperature for 5 minutes, then add an equal volume of pre-warmed DPBS.
(8) Count the cells and centrifuge again at room temperature at 100 g for 10 minutes.
(9) Resuspend the cells in an appropriate volume of T-cell culture medium (X-Vivo, 5% AB serum, 400 IU IL-2) at 2 × 10⁶ cells/mL.
(10) Remove the antibody incubation solution from the T25 flask, add the T-cell suspension, and culture normally at 37 °C for approximately 48 hours.

### 4. Preparation of CAR-T cells

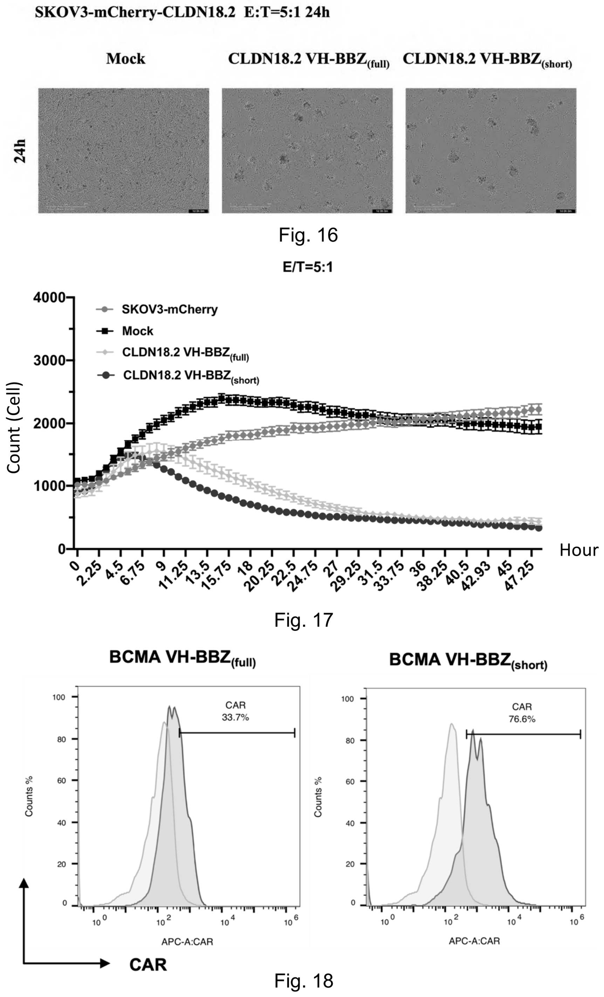

(1) Pre-coat the T-cell culture plate one day in advance and incubate overnight at 4 °C. A 24-well plate is used, and each well is supplemented with 0.5 mL DPBS and 10 µL RetroNectin (1 mg/mL).
(2) Remove the RetroNectin solution.
(3) Add 1 mL of 2% BSA solution to each well and block at 37 °C for 30 minutes.
(4) Remove the blocking solution and wash once with DPBS.
(5) To each well (non-adherent plate), add 200 µL of 400× virus and 300 µL X-Vivo complete medium, then centrifuge at 32 °C, 1000 g (centrifuge acceleration: up 2, down 2) for 2 hours.
(6) Add 2-2.5 × 10⁶ T cells activated for approximately 48 hours into each well, bring the volume to 1 mL per well with X-Vivo complete medium, and centrifuge at 32 °C, 300 g (centrifuge acceleration: up 2, down 2) for 15 minutes.
   The CAR-T cells synthesized in this embodiment are referred to as BCMA VH-BBZ(short), and the control CAR-T cells are referred to as BCMA VH-BBZ(full).
(7) Culture normally at 37 °C for approximately 72 hours. Perform flow cytometry to detect CAR positive expression rates in the experimental group and the control group, as shown in Fig. 18. The results show that the positive expression rate of BCMA VH-BBZ(short) is 76.6%, whereas that of BCMA VH-BBZ(full) is 33.7%.

### EMBODIMENT 6

This embodiment provides an in vitro killing assay for the CAR-T cells synthesized in Embodiment 5.
(1) 96-well plate killing assay: Tumor cells were seeded at 1 × 10⁴ cells per well. The CAR-T cells synthesized in Embodiment 5 were co-cultured with the tumor cells at effector-to-target ratios of 2.5:1, 5:1 and 10:1, respectively. Each effector-to-target ratio contained three replicate wells. Mock T cells were used as the control group. After approximately 24 hours of co-culture, the culture supernatant was collected to measure LDH for calculating the tumor lysis rate.
   In this embodiment, the human multiple myeloma cell line MM1S-mCherry was used, which is a broadly representative tumor cell model.
(2) A live-cell imaging system was also used to continuously monitor the in vitro killing activity of CAR-T cells in the experimental and control groups. CAR-T cells and tumor cells were co-cultured on plates at effector-to-target ratios of 2.5:1, 5:1 and 10:1, with three replicate wells for each effector-to-target ratio.

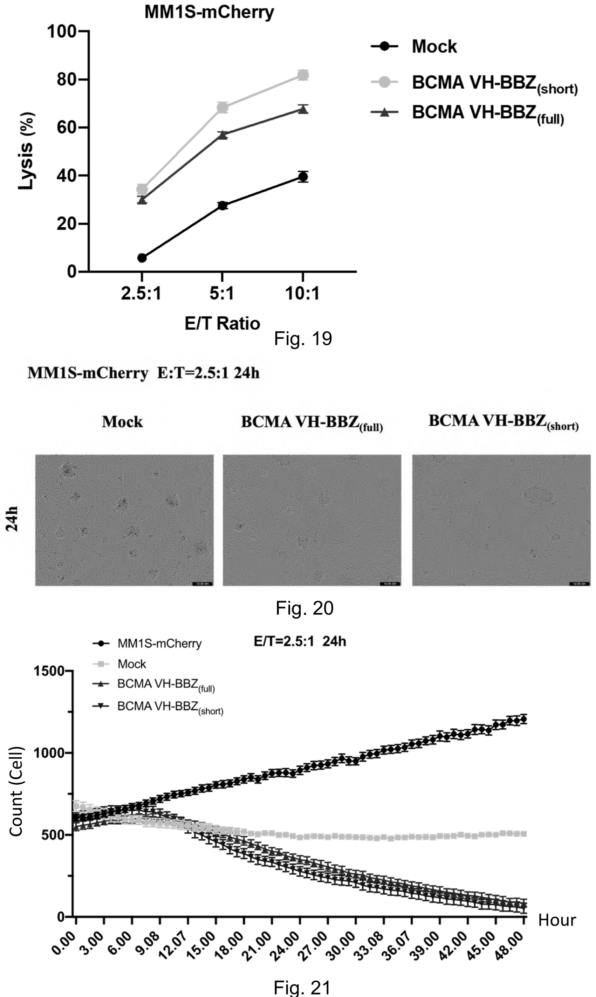

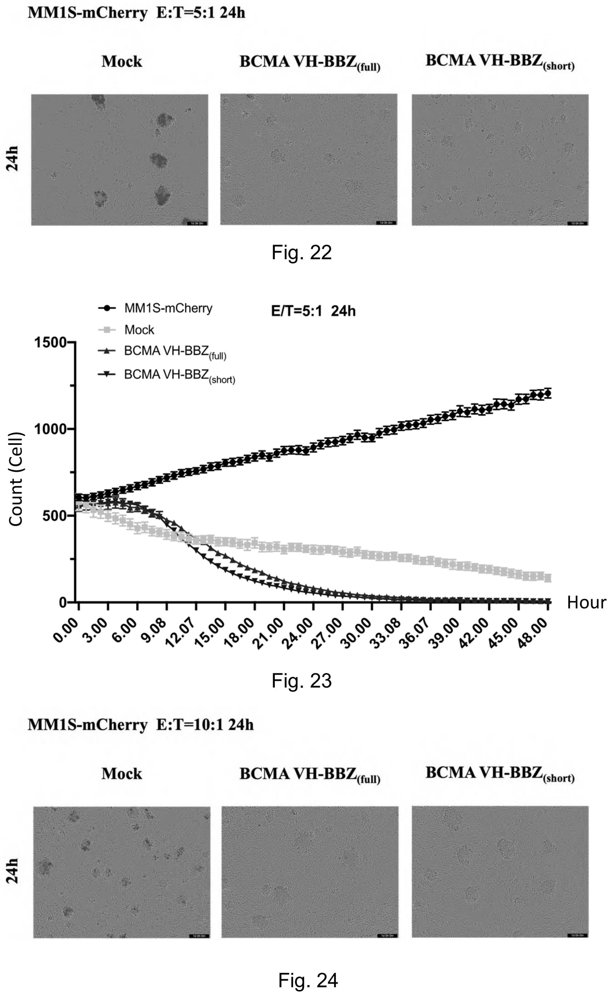

The experimental results are shown in Figs. 19-25.

Fig. 19 shows that both the CAR-T cells synthesized in Embodiment 5 and the full-length control CAR-T cells exerted effective killing against BCMA-positive tumor cells. However, the miniaturized BCMA VH-BBZ(short) CAR-T cells exhibited distinctly stronger tumor-killing ability at higher effector-to-target ratios. At an effector-to-target ratio of E:T = 5:1, the BCMA VH-BBZ(short) CAR-T cells showed slightly better killing activity than the BCMA VH-BBZ(full) CAR-T cells; and at an effector-to-target ratio of E:T = 10:1, the killing activity was markedly superior to that of the BCMA VH-BBZ(full) CAR-T cells.

Specifically, Figs. 20-25 show the continuous killing effects under different effector-to-target ratios. Taking E:T = 10:1 as an example (E represents effector cells; T represents target tumor cells), during the first 7 hours of continuous killing, the killing activity of BCMA VH-BBZ(short) CAR-T cells and BCMA VH-BBZ(full) CAR-T cells was not markedly different; however, after 7 hours, the killing effect of BCMA VH-BBZ(short) CAR-T cells gradually surpassed that of BCMA VH-BBZ(full) CAR-T cells. Taking E:T = 5:1 as another example, during the first 9 hours of continuous killing, the difference in killing activity between BCMA VH-BBZ(short) CAR-T cells and BCMA VH-BBZ(full) CAR-T cells was not prominent; however, after 9 hours, the killing effect of BCMA VH-BBZ(short) CAR-T cells gradually became superior. These results indicate that a single CD3ζ ITAM domain (CD3ζ ITAM1) is sufficient to activate downstream signalling of a T cell, enabling CAR-T cells to exert effective tumor killing while avoiding excessive activation of downstream T-cell signalling that may damage CAR-T cells, thereby improving the persistence of tumor-killing activity.

Embodiments 1-6 provide different examples of miniaturized second-generation CAR structures, in which the smallest CAR contains 281 amino acids and the largest CAR contains 294 amino acids.

### EMBODIMENT 7

This embodiment provides examples of three additional preparation methods for CAR-T cells.

### 1. Construction of the CAR expression vector

(1) The CAR structure comprises an extracellular HER2 nanobody; a CD28 hinge region and a CD28 transmembrane region, or a CD28 hinge region and a CD3 transmembrane region, or a CD8 hinge region and a CD3 transmembrane region; an intracellular co-stimulatory molecule (4-1BB); and a portion of a CD3ζ signalling domain (CD3ζ ITAM1) / a full-length CD3ζ signalling domain (control). The structural fragments were prepared through gene synthesis.
   It is to be understood that the extracellular nanobody may be selected from various nanobodies targeting antigens of solid tumors or hematologic tumors. The nanobodies illustrated in the following embodiments are provided only as examples and should not be construed as exclusive. In this embodiment, a nanobody targeting HER2 is used as an example.
(2) The Pwpxld vector and the CAR structural fragment were each digested with the restriction endonucleases EcoRI and BamHI. After ligation using T4 ligase, transformation and screening were performed. Positive clones obtained were subjected to sequencing analysis, ultimately yielding the Her2-28H+28TM-VH-BBZ(short)-PwpxId, Her2-28H+3TM-VH-BBZ(short)-Pwpxld, and Her2-8H+3TM-VH-BBZ(short)-PwpxId expression vectors.

The CAR structure of the present invention is shown in Fig. 1.

The sequences of the respective fragments synthesized or amplified in this embodiment are shown in Tables 4-6.

**Table 4: Her2-28H+28TM-VH-BBZ(short)**

| NO. | Sequence | |
|---|---|---|
| 9 | Her2 CAR | ATGGCCTTACCAGTGACCGCCTTGCTCCTGCCGCTGGCCTTGCTGCTCCAC GCCGCCAGGCCGGAGGTGCAGCTGGTGGAGAGCGGGGGGGGACTGGTGC AGGCAGGAGGAAGCCTGAGACTGAGCTGTGCCGCAAGCGGGATTACATTTA GTATCAACACAATGGGATGGTACAGGCAGGCTCCTGGCAAGCAGAGAGAGC TGGTGGCTCTGATTTCAAGCATCGGAGACACCTACTACGCCGACTCTGTGA AAGGACGGTTCACCATCAGCAGAGACAACGCAAAGAACACCGTCTACCTGC AGATGAACAGCCTGAAACCTGAAGACACAGCAGTGTACTATTGCAAAAGGTT CCGAACCGCTGCTCAGGGCACTGACTACTGGGGCCAGGGCACCCAGGTGA CCGTGTCCAGCATTGAAGTTATGTATCCTCCTCCTTACCTAGACAATGAGAA GAGCAATGGAACCATTATCCATGTGAAAGGGAAACACCTTTGTCCAAGTCCC CTATTTCCCGGACCTTCTAAGCCCTTTTGGGTGCTGGTGGTTGGTGGA GTCCTGGCTTGCTATAGCTTGCTAGTAACAGTGGCCTTTATTATTTTCTGGGT GAAACGGGGCAGAAAGAAACTCCTGTATATATTCAAACAACCATTTATGAGA CCAGTACAAACTACTCAAGAGGAAGATGGCTGTAGCTGCCGATTTCCAGAA |
| | | GAAGAAGAAGGAGGATGTGAACTGGCCCCCGCGTACCAGCAGGGCCAGAA CCAGCTCTATAACGAGCTCAATCTAGGACGAAGAGAGGAGTACGATGTTTTG CACATGCAGGCCCTGCCCCCTCGCTGA (281 amino acids) |
| 10 | CD28H +CD28 TM | ATTGAAGTTATGTATCCTCCTCCTTACCTAGACAATGAGAAGAGCAATGGAA CCATTATCCATGTGAAAGGGAAACACCTTTGTCCAAGTCCCCTATTTCCCGG ACCTTCTAAGCCCTTTTGGGTGCTGGTGGTGGTTGGTGGAGTCCTGGCTTG CTATAGCTTGCTAGTAACAGTGGCCTTTATTATTTTCTGGGTG (66 amino acids) |

**Table 5 Her2-28H+3TM-VH-BBZ(short)**

| NO. | Sequence | |
|---|---|---|
| 11 | Her2 CAR | ATGGCCTTACCAGTGACCGCCTTGCTCCTGCCGCTGGCCTTGCTGCTCCAC GCCGCCAGGCCGGAGGTGCAGCTGGTGGAGAGCGGGGGGGGACTGGTGC AGGCAGGAGGAAGCCTGAGACTGAGCTGTGCCGCAAGCGGGATTACATTTA GTATCAACACAATGGGATGGTACAGGCAGGCTCCTGGCAAGCAGAGAGAGC TGGTGGCTCTGATTTCAAGCATCGGAGACACCTACTACGCCGACTCTGTGA AAGGACGGTTCACCATCAGCAGAGACAACGCAAAGAACACCGTCTACCTGC AGATGAACAGCCTGAAACCTGAAGACACAGCAGTGTACTATTGCAAAAGGTT CCGAACCGCTGCTCAGGGCACTGACTACTGGGGCCAGGGCACCCAGGTGA CCGTGTCCAGCATTGAAGTTATGTATCCTCCTCCTTACCTAGACAATGAGAA GAGCAATGGAACCATTATCCATGTGAAAGGGAAACACCTTTGTCCAAGTCCC CTATTTCCCGGACCTTCTAAGCCCCTCTGCTACCTGCTGGATGGAATCCTCT TCATCTATGGTGTCATTCTCACTGCCTTGTTCCTGAAACGGGGCAGAAAGAA ACTCCTGTATATATTCAAACAACCATTTATGAGACCAGTACAAACTACTCAAG AGGAAGATGGCTGTAGCTGCCGATTTCCAGAAGAAGAAGAAGGAGGATGTG AACTGGCCCCCGCGTACCAGCAGGGCCAGAACCAGCTCTATAACGAGCTCA ATCTAGGACGAAGAGAGGAGTACGATGTTTTGCACATGCAGGCCCTGCCCC CTCGCTGA (275 amino acids) |
| 12 | CD28H +CD3T M | ATTGAAGTTATGTATCCTCCTCCTTACCTAGACAATGAGAAGAGCAATGGAA CCATTATCCATGTGAAAGGGAAACACCTTTGTCCAAGTCCCCTATTTCCCGG ACCTTCTAAGCCCCTCTGCTACCTGCTGGATGGAATCCTCTTCATCTATGGT GTCATTCTCACTGCCTTGTTCCTG (60 amino acids) |

**Table 6. Her2-8H+3TM-VH-BBZ(short)**

| NO. | Sequence | |
|---|---|---|
| 13 | Her2 CAR | ATGGCCTTACCAGTGACCGCCTTGCTCCTGCCGCTGGCCTTGCTGCTCCAC GCCGCCAGGCCGGAGGTGCAGCTGGTGGAGAGCGGGGGGGGACTGGTGC AGGCAGGAGGAAGCCTGAGACTGAGCTGTGCCGCAAGCGGGATTACATTTA GTATCAACACAATGGGATGGTACAGGCAGGCTCCTGGCAAGCAGAGAGAGC TGGTGGCTCTGATTTCAAGCATCGGAGACACCTACTACGCCGACTCTGTGAA AGGACGGTTCACCATCAGCAGAGACAACGCAAAGAACACCGTCTACCTGCA GATGAACAGCCTGAAACCTGAAGACACAGCAGTGTACTATTGCAAAAGGTTC CGAACCGCTGCTCAGGGCACTGACTACTGGGGCCAGGGCACCCAGGTGAC CGTGTCCAGCACCACGACGCCAGCGCCGCGACCACCAACACCGGCGCCCA CCATCGCGTCGCAGCCCCTGTCCCTGCGCCCAGAGGCGTGCCGGCCAGCG GCGGGGGGCGCAGTGCACACGAGGGGGCTGGACTTCGCCTGTGATCTCTG CTACCTGCTGGATGGAATCCTCTTCATCTATGGTGTCATTCTCACTGCCTTGT |
| | | TCCTGAAACGGGGCAGAAAGAAACTCCTGTATATATTCAAACAACCATTTAT GAGACCAGTACAAACTACTCAAGAGGAAGATGGCTGTAGCTGCCGATTTCC AGAAGAAGAAGAAGGAGGATGTGAACTGGCCCCCGCGTACCAGCAGGGCC AGAACCAGCTCTATAACGAGCTCAATCTAGGACGAAGAGAGGAGTACGATG TTTTGCACATGCAGGCCCTGCCCCCTCGCTGA (281 amino acids) |
| 14 | CD8H+ CD3T M | ACCACGACGCCAGCGCCGCGACCACCAACACCGGCGCCCACCATCGCGTC GCAGCCCCTGTCCCTGCGCCCAGAGGCGTGCCGGCCAGCGGCGGGGGGC GCAGTGCACACGAGGGGGCTGGACTTCGCCTGTGATCTCTGCTACCTGCTG GATGGAATCCTCTTCATCTATGGTGTCATTCTCACTGCCTTGTTCCTG (66 amino acids) |

### 2. Lentiviral packaging

(1) Thaw and culture 293T cells; when the confluency reaches approximately 80%, proceed with viral packaging.
(2) Add chloroquine solution to the culture medium (DMEM + 10% FBS) to a final concentration of 100 µmol/L.
(3) The viral packaging system (per 10-cm culture dish) comprises: 50 µL CaCl₂, 20 µg of the constructed core plasmid, 10 µg Pspax2, 5 µg PMD2.0G, 500 µL nuclease-free water, and 500 µL 2× BBS.
(4) After approximately 10 hours, replace with fresh pre-warmed culture medium (DMEM + 10% FBS).
(5) Collect the viral supernatants at 48 and 72 hours of culture; filter using a 0.22-µm membrane to remove impurities.
(6) Ultracentrifuge, resuspend the concentrated virus in DPBS at 1/400 of the original volume of the viral supernatant, aliquot according to subsequent usage requirements, and store at -80 °C for later use.

### 3. Isolation of peripheral blood cells

(1) Pre-coat T-cell culture flasks (T25) one day in advance and incubate overnight at 4 °C. The T-cell culture medium contains anti-human CD3 and CD28 monoclonal antibodies: 3 mL DPBS, 4 µg/mL CD3, and 2 µg/mL CD28.
(2) Collect an appropriate amount of freshly drawn blood from a healthy volunteer.
(3) Add 5 mL Ficoll to a 15-mL BD tube, and gently layer 5 mL of fresh blood on top.
(4) Centrifuge at room temperature at 1000 g (centrifuge acceleration: up 1, down 1) for 30 minutes.
(5) After centrifugation, carefully aspirate the middle (white) layer containing peripheral blood lymphocytes.
(6) Wash with three volumes of pre-warmed DPBS, centrifuge at 300 g for 10 minutes.
(7) Optionally, add 5 mL red blood cell lysis buffer and lyse at room temperature for 5 minutes, then add an equal volume of pre-warmed DPBS.
(8) Count the cells and centrifuge again at room temperature at 100 g for 10 minutes.
(9) Resuspend the cells in an appropriate volume of T-cell culture medium (X-Vivo, 5% AB serum, 400 IU IL-2) at 2 × 10⁶ cells/mL.
(10) Remove the antibody incubation solution from the T25 flask, add the T-cell suspension, and culture normally at 37 °C for approximately 48 hours.

### 4. Preparation of CAR-T cells

(1) Pre-coat the T-cell culture plate one day in advance and incubate overnight at 4 °C. A 24-well plate is used, and each well is supplemented with 0.5 mL DPBS and 10 µL RetroNectin (1 mg/mL).
(2) Remove the RetroNectin solution.
(3) Add 1 mL of 2% BSA solution to each well and block at 37 °C for 30 minutes.
(4) Remove the blocking solution and wash once with DPBS.
(5) To each well (non-adherent plate), add 200 µL of 400× virus and 300 µL X-Vivo complete medium, then centrifuge at 32 °C, 1000 g (centrifuge acceleration: up 2, down 2) for 2 hours.
(6) Add 2-2.5 × 10⁶ T cells activated for approximately 48 hours into each well, bring the volume to 1 mL per well with X-Vivo complete medium, and centrifuge at 32 °C, 300 g (centrifuge acceleration: up 2, down 2) for 15 minutes.
   The miniaturized CAR-T cells synthesized in this embodiment are referred to as Her2-28H+28TM-VH-BBZ(short), Her2-28H+3TM-VH-BBZ(short), or Her2-8H+3TM-VH-BBZ(short). The full-length control CAR-T cells are referred to as Her2 VH-BBZ(full), in which both the hinge region and the transmembrane region are derived from CD8, also denoted as Her2-8H+8TM-VH-BBZ(full).
(7) Culture normally at 37 °C for approximately 72 hours and then perform the in vitro killing assay.

### EMBODIMENT 8

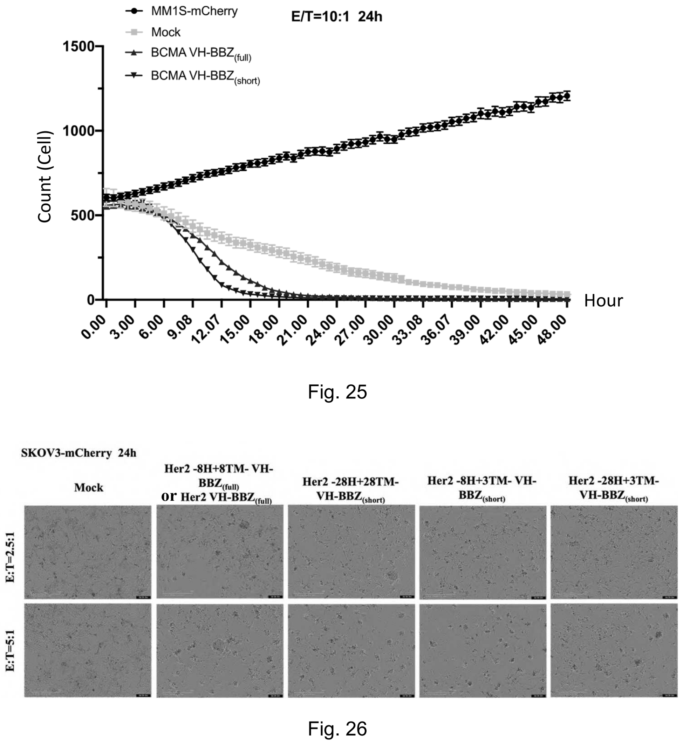

This embodiment provides an in vitro killing assay for the CAR-T cells synthesized in Embodiment 7.
(1) 96-well plate killing assay: Tumor cells were seeded at 1 × 10⁴ cells per well. The CAR-T cells synthesized in Embodiment 1 were co-cultured with the tumor cells at effector-to-target ratios (effector cells: target cells = CAR-T : tumor cells) of 2.5:1 and 5:1, respectively. Each effector-to-target ratio contained three replicate wells, and mock T cells (Mock) were used as the control.
   In this embodiment, the human ovarian cancer cell line SKOV3-mCherry was used, which is a broadly representative tumor cell model.
(2) A live-cell imaging system was used to record a single-time-point image of the killing activity of CAR-T cells in each experimental group and the control group at 24 hours of coincubation. The experimental results are shown in Fig. 26.

Fig. 26 shows that all miniaturized CAR-T cells synthesized in Embodiment 7, as well as the full-length control CAR-T cells, exert effective killing against HER2-positive tumor cells. However, compared with the control Her2 VH-BBZ(full) CAR-T cells (hinge region and transmembrane region both derived from CD8, also denoted as Her2-8H+8TM-VH-BBZ(full)), the Her2-28H+28TM-VH-BBZ(short) and Her2-8H+3TM-VH-BBZ(short) CAR-T cells exhibit significantly stronger tumor-killing ability. The Her2-28H+3TM-VH-BBZ(short) CAR-T cells also show slightly better tumor-killing activity than Her2 VH-BBZ(full) (hinge region and transmembrane region derived from CD8, also denoted as Her2-8H+8TM-VH-BBZ(full)). Specifically, these results illustrate that the use of hinge regions and transmembrane regions derived from different molecules can all confer the advantageous killing properties of the novel miniaturized CAR molecule, sufficiently activating downstream signalling of a T cell to promote effective tumor-killing activity by CAR-T cells.

### EMBODIMENT 9

This embodiment provides an example of a preparation method for another type of CAR-T cell.

### 1. Construction of the CAR expression vector

(1) The CAR structure comprises an extracellular HER2 nanobody (VH) / HER2 singlechain antibody (scFv) (control), a CD8 hinge region, a CD8 transmembrane region, an intracellular co-stimulatory molecule (4-1BB), and a portion of a CD3ζ signalling domain (CD3ζ ITAM1)/ a full-length CD3ζ signalling domain (control). The structural fragments were prepared through gene synthesis.
   In this embodiment, CAR molecules constructed with different HER2-targeting designs are used as examples.
(2) The Pwpxld vector and the CAR structural fragment were each digested with the restriction endonucleases EcoRI and BamHI. After ligation using T4 ligase, transformation and screening were performed. Positive clones obtained were subjected to sequencing analysis, ultimately yielding the Her2 scFv-BBZ(short)-Pwpxld expression vector, the Her2 scFv-BBZ(full)-Pwpxld control vector (containing full-length CD3ζ), the Her2 VH-BBZ(short)-Pwpxld expression vector, and the Her2 VH-BBZ(full)-Pwpxld control vector (containing full-length CD3ζ).

The CAR structure of the present invention is shown in Fig. 1.

The sequences of the respective fragments synthesized or amplified in this embodiment are shown in the following tables.

**Table 7: Her2 scFv-BBZ(short)**

| NO. | Sequence | |
|---|---|---|
| 15 | Her2 CAR | ATGGCCTTACCAGTGACCGCCTTGCTCCTGCCGCTGGCCTTGCTGCTCCAC GCCGCCAGGCCGATGCAGGTACAACTGCAGCAGTCAGGACCTGAACTGAA GAAGCCTGGAGAGACAGTCAAGATCTCCTGCAAGGCCTCTGGGTATCCTTT CACAAACTATGGAATGAACTGGGTGAAGCAGGCTCCAGGACAGGGTTTAAA GTGGATGGGCTGGATTAACACCTCCACTGGAGAGTCAACATTTGCTGATGA CTTCAAGGGACGGTTTGACTTCTCTTTGGAAACCTCTGCCAACACTGCCTAT TTGCAGATCAACAACCTCAAAAGTGAAGACTCGGCTACATATTTCTGTGCAA GATGGGAGGTTTACCACGGCTACGTTCCTTACTGGGGCCAAGGGACCACG GTCACCGTTTCCTCTGGCGGTGGCGGTTCTGGTGGCGGTGGCTCCGGCGG TGGCGGTTCTGACATCCAGCTGACCCAGTCTCACAAATTCCTGTCCACTTCA GTAGGAGACAGGGTCAGCATCACCTGCAAGGCCAGTCAGGATGTGTATAAT GCTGTTGCCTGGTATCAACAGAAACCAGGACAATCTCCTAAACTTCTGATTT ACTCGGCATCCTCCCGGTACACTGGAGTCCCTTCTCGCTTCACTGGCAGTG GCTCTGGGCCGGATTTCACTTTCACCATCAGCAGTGTGCAGGCTGAAGACC TGGCAGTTTATTTCTGTCAGCAACATTTTCGTACTCCATTCACGTTCGGCTCG GGGACAAAATTGGAGATCAAAACCACGACGCCAGCGCCGCGACCACCAAC |
| | | ACCGGCGCCCACCATCGCGTCGCAGCCCCTGTCCCTGCGCCCAGAGGCGT GCCGGCCAGCGGCGGGGGGCGCAGTGCACACGAGGGGGCTGGACTTCGC CTGTGATATCTACATCTGGGCGCCCCTGGCCGGGACTTGTGGGGTCCTTCT CCTGTCACTGGTTATCACCAAACGGGGCAGAAAGAAACTCCTGTATATATTC AAACAACCATTTATGAGACCAGTACAAACTACTCAAGAGGAAGATGGCTGTA GCTGCCGATTTCCAGAAGAAGAAGGAGGATGTGAACTGGCCCCCGCG TACCAGCAGGGCCAGAACCAGCTCTATAACGAGCTCAATCTAGGACGAAGA GAGGAGTACGATGTTTTGCACATGCAGGCCCTGCCCCCTCGCTGA (405 amino acids) |
| 16 | HER2 Single Chain Antibod y | ATGCAGGTACAACTGCAGCAGTCAGGACCTGAACTGAAGAAGCCTGGAGA GACAGTCAAGATCTCCTGCAAGGCCTCTGGGTATCCTTTCACAAACTATGG AATGAACTGGGTGAAGCAGGCTCCAGGACAGGGTTTAAAGTGGATGGGCT GGATTAACACCTCCACTGGAGAGTCAACATTTGCTGATGACTTCAAGGGAC GGTTTGACTTCTCTTTGGAAACCTCTGCCAACACTGCCTATTTGCAGATCAA CAACCTCAAAAGTGAAGACTCGGCTACATATTTCTGTGCAAGATGGGAGGT TTACCACGGCTACGTTCCTTACTGGGGCCAAGGGACCACGGTCACCGTTTC CTCTGGCGGTGGCGGTTCTGGTGGCGGTGGCTCCGGCGGTGGCGGTTCT GACATCCAGCTGACCCAGTCTCACAAATTCCTGTCCACTTCAGTAGGAGAC AGGGTCAGCATCACCTGCAAGGCCAGTCAGGATGTGTATAATGCTGTTGCC TGGTATCAACAGAAACCAGGACAATCTCCTAAACTTCTGATTTACTCGGCAT CCTCCCGGTACACTGGAGTCCCTTCTCGCTTCACTGGCAGTGGCTCTGGG CCGGATTTCACTTTCACCATCAGCAGTGTGCAGGCTGAAGACCTGGCAGTT TATTTCTGTCAGCAACATTTTCGTACTCCATTCACGTTCGGCTCGGGGACAA AATTGGAGATCAAA (242 amino acids) |

**Table 8. Her2 scFV-BBZ(full)**

| NO. | Sequence | |
|---|---|---|
| 17 | Her2 CAR | ATGGCCTTACCAGTGACCGCCTTGCTCCTGCCGCTGGCCTTGCTGCTCCAC GCCGCCAGGCCGATGCAGGTACAACTGCAGCAGTCAGGACCTGAACTGAA GAAGCCTGGAGAGACAGTCAAGATCTCCTGCAAGGCCTCTGGGTATCCTTT CACAAACTATGGAATGAACTGGGTGAAGCAGGCTCCAGGACAGGGTTTAAA GTGGATGGGCTGGATTAACACCTCCACTGGAGAGTCAACATTTGCTGATGA CTTCAAGGGACGGTTTGACTTCTCTTTGGAAACCTCTGCCAACACTGCCTAT TTGCAGATCAACAACCTCAAAAGTGAAGACTCGGCTACATATTTCTGTGCAA GATGGGAGGTTTACCACGGCTACGTTCCTTACTGGGGCCAAGGGACCACG GTCACCGTTTCCTCTGGCGGTGGCGGTTCTGGTGGCGGTGGCTCCGGCGG TGGCGGTTCTGACATCCAGCTGACCCAGTCTCACAAATTCCTGTCCACTTCA GTAGGAGACAGGGTCAGCATCACCTGCAAGGCCAGTCAGGATGTGTATAAT GCTGTTGCCTGGTATCAACAGAAACCAGGACAATCTCCTAAACTTCTGATTT ACTCGGCATCCTCCCGGTACACTGGAGTCCCTTCTCGCTTCACTGGCAGTG GCTCTGGGCCGGATTTCACTTTCACCATCAGCAGTGTGCAGGCTGAAGACC TGGCAGTTTATTTCTGTCAGCAACATTTTCGTACTCCATTCACGTTCGGCTCG GGGACAAAATTGGAGATCAAAACCACGACGCCAGCGCCGCGACCACCAAC ACCGGCGCCCACCATCGCGTCGCAGCCCCTGTCCCTGCGCCCAGAGGCGT GCCGGCCAGCGGCGGGGGGCGCAGTGCACACGAGGGGGCTGGACTTCGC CTGTGATATCTACATCTGGGCGCCCCTGGCCGGGACTTGTGGGGTCCTTCT CCTGTCACTGGTTATCACCAAACGGGGCAGAAAGAAACTCCTGTATATATTC AAACAACCATTTATGAGACCAGTACAAACTACTCAAGAGGAAGATGGCTGTA GCTGCCGATTTCCAGAAGAAGAAGAAGGAGGATGTGAACTGGCCCCCGCG TACCAGCAGGGCCAGAACCAGCTCTATAACGAGCTCAATCTAGGACGAAGA GAGGAGTACGATGTTTTGGACAAGAGACGTGGCCGGGACCCTGAGATGGG |
| | | GGGAAAGCCGCAGAGAAGGAAGAACCCTCAGGAAGGCCTGTACAATGAAC TGCAGAAAGATAAGATGGCGGAGGCCTACAGTGAGATTGGGATGAAAGGC GAGCGCCGGAGGGGCAAGGGGCACGATGGCCTTTACCAGGGTCTCAGTAC AGCCACCAAGGACACCTACGACGCCCTTCACATGCAGGCCCTGCCCCCTCG CTGA (476 amino acids) |
| 18 | CD3ζ full ength | GCCCCCGCGTACCAGCAGGGCCAGAACCAGCTCTATAACGAGCTCAATCT AGGACGAAGAGAGGAGTACGATGTTTTGGACAAGAGACGTGGCCGGGACC CTGAGATGGGGGGAAAGCCGCAGAGAAGGAAGAACCCTCAGGAAGGCCT GTACAATGAACTGCAGAAAGATAAGATGGCGGAGGCCTACAGTGAGATTGG GATGAAAGGCGAGCGCCGGAGGGGCAAGGGGCACGATGGCCTTTACCAG GGTCTCAGTACAGCCACCAAGGACACCTACGACGCCCTTCACATGCAGGC CCTGCCCCCTCGC (104 amino acids) |

### 2. Lentiviral packaging

(1) Thaw and culture 293T cells; when the confluency reaches approximately 80%, proceed with viral packaging.
(2) Add chloroquine solution to the culture medium (DMEM + 10% FBS) to a final concentration of 100 µmol/L.
(3) The viral packaging system (per 10-cm culture dish) comprises: 50 µL CaCl₂, 20 µg of the constructed core plasmid, 10 µg Pspax2, 5 µg PMD2.0G, 500 µL nuclease-free water, and 500 µL 2× BBS.
(4) After approximately 10 hours, replace with fresh pre-warmed culture medium (DMEM + 10% FBS).
(5) Collect the viral supernatants at 48 and 72 hours of culture; filter using a 0.22-µm membrane to remove impurities.
(6) Ultracentrifuge, resuspend the concentrated virus in DPBS at 1/400 of the original volume of the viral supernatant, aliquot according to subsequent usage requirements, and store at -80 °C for later use.

### 3. Isolation of peripheral blood cells

(1) Pre-coat T-cell culture flasks (T25) one day in advance and incubate overnight at 4 °C. The T-cell culture medium contains anti-human CD3 and CD28 monoclonal antibodies: 3 mL DPBS, 4 µg/mL CD3, and 2 µg/mL CD28.
(2) Collect an appropriate amount of freshly drawn blood from a healthy volunteer.
(3) Add 5 mL Ficoll to a 15-mL BD tube, and gently layer 5 mL of fresh blood on top.
(4) Centrifuge at room temperature at 1000 g (centrifuge acceleration: up 1, down 1) for 30 minutes.
(5) After centrifugation, carefully aspirate the middle (white) layer containing peripheral blood lymphocytes.
(6) Wash with three volumes of pre-warmed DPBS, centrifuge at 300 g for 10 minutes.
(7) Optionally, add 5 mL red blood cell lysis buffer and lyse at room temperature for 5 minutes, then add an equal volume of pre-warmed DPBS.
(8) Count the cells and centrifuge again at room temperature at 100 g for 10 minutes.
(9) Resuspend the cells in an appropriate volume of T-cell culture medium (X-Vivo, 5% AB serum, 400 IU IL-2) at 2 × 10⁶ cells/mL.
(10) Remove the antibody incubation solution from the T25 flask, add the T-cell suspension, and culture at 37 °C for approximately 48 hours.

### 4. Preparation of CAR-T cells

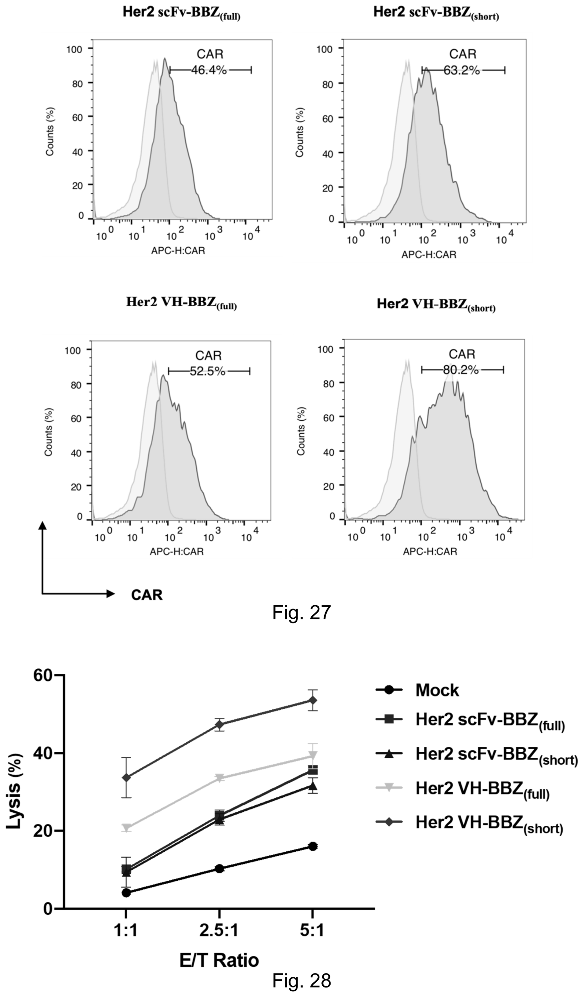

(1) Pre-coat the T-cell culture plate one day in advance and incubate overnight at 4 °C. A 24-well plate is used, and each well is supplemented with 0.5 mL DPBS and 10 µL RetroNectin (1 mg/mL).
(2) Remove the RetroNectin solution.
(3) Add 1 mL of 2% BSA solution to each well and block at 37 °C for 30 minutes.
(4) Remove the blocking solution and wash once with DPBS.
(5) To each well (non-adherent plate), add 200 µL of 400× virus and 300 µL X-Vivo complete medium, then centrifuge at 32 °C, 1000 g (centrifuge acceleration: up 2, down 2) for 2 hours.
(6) Add 2-2.5 × 10⁶ T cells activated for approximately 48 hours into each well, bring the volume to 1 mL per well with X-Vivo complete medium, and centrifuge at 32 °C, 300 g (centrifuge acceleration: up 2, down 2) for 15 minutes.
   The miniaturized CAR-T cells synthesized in this embodiment are referred to as Her2 scFv-BBZ(full) (containing full-length CD3ζ), Her2 scFv-BBZ(short), Her2 VH-BBZ(full) (containing full-length CD3ζ) and Her2 VH-BBZ(short).
(7) Culture normally at 37 °C for approximately 72 hours. Perform flow cytometry to detect CAR positive expression rates in the experimental and control groups, as shown in Fig. 27. The results show that the positive expression rate is 46.4% for Her2 scFv-BBZ(full), 63.2% for Her2 scFv-BBZ(short), 52.5% for Her2 VH-BBZ(full), and 80.2% for Her2 VH-BBZ(short).

### EMBODIMENT 10

This embodiment provides an in vitro killing assay for the CAR-T cells synthesized in Embodiment 9.
(1) 96-well plate killing assay: Tumor cells were seeded at 1 × 10⁴ cells per well. The CAR-T cells synthesized in Embodiment 1 were co-cultured with the tumor cells at effector-to-target ratios of 1:1, 2.5:1 and 5:1. Each effector-to-target ratio contained three replicate wells, and mock T cells (Mock) were used as the control.
   In this embodiment, the human ovarian cancer cell line SKOV3-mCherry was used, which is a broadly representative tumor model.
(2) A live-cell imaging system was used to continuously monitor the in vitro killing activity of CAR-T cells in the experimental group and the control group.
(3) After approximately 24 hours of co-culture, the culture supernatant was collected to measure LDH for calculating the tumor lysis rate.

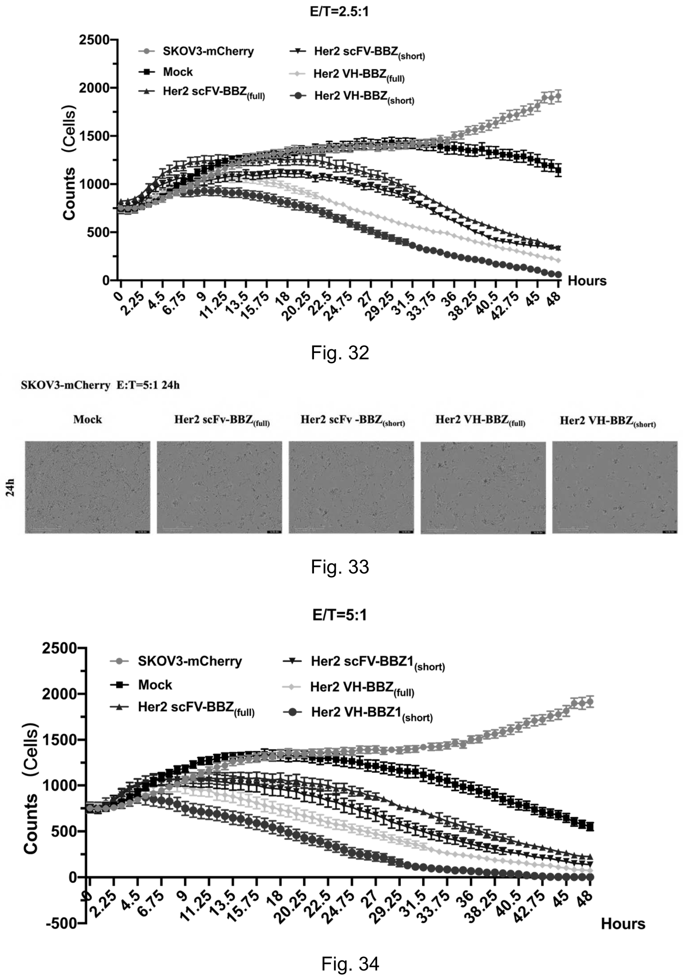

The experimental results are shown in Figs. 28-34.

Fig. 28 shows that all CAR-T cells synthesised in Embodiment 9, each expressing CAR molecules with different structural designs, exert effective killing against HER2-positive tumor cells. However, the Her2 VH-BBZ(short) CAR-T cells exhibit clearly superior tumor-killing ability. At effector-to-target ratios of E:T = 1:1, 2.5:1 and 5:1, the killing activity of the Her2 VH-BBZ(short) CAR-T cells is significantly better than that of Her2 scFv-BBZ(full), Her2 scFv-BBZ(short) and Her2 VH-BBZ(full).

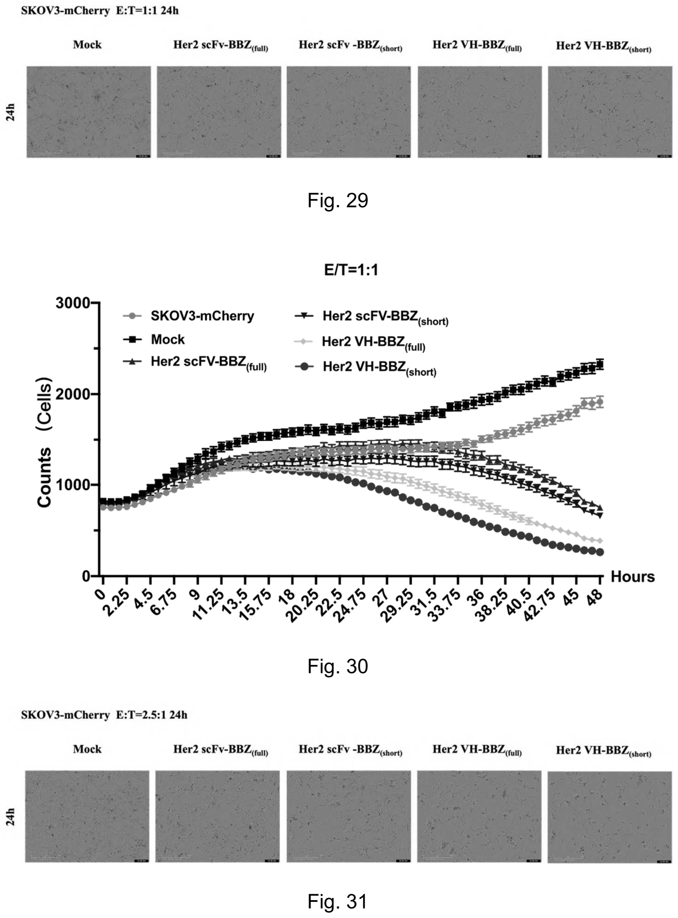

Specifically, Figs. 29-34 show the continuous killing effects at different effector-to-target ratios. At E:T = 1:1, 2.5:1 and 5:1 (E representing effector cells and T representing target tumor cells), after 24 hours of continuous killing, the tumor-killing activity of Her2 VH-BBZ(short) CAR-T cells is consistently superior to that of Her2 scFv-BBZ(full), Her2 scFv-BBZ(short) and Her2 VH-BBZ(full), with Her2 scFv-BBZ(full) showing the weakest killing effect. At E:T = 1:1, Her2 VH-BBZ(short) CAR-T cells begin to exhibit killing activity at approximately 16 hours; at E:T = 2.5:1, killing begins at approximately 9 hours; and at E:T = 5:1, killing begins at approximately 6 hours. These onset times are earlier than those of Her2 scFv-BBZ(full), Her2 scFv-BBZ(short) and Her2 VH-BBZ(full), with Her2 scFv-BBZ(full) showing the latest onset. These results indicate that the length of a CAR molecule correlates with both the strength and the onset time of CAR-T killing activity, with shorter CAR molecules exhibiting stronger tumor-killing activity and earlier onset of tumor-killing effects.

The embodiments of the present invention are described above with reference to the accompanying drawings, but the present invention is not limited to the aforementioned specific embodiments. The aforementioned embodiments are merely illustrative and not limiting. For those of ordinary skill in the art, many forms can be made under the teaching of present invention without departing from the spirit of the present invention and the scope of the claims, all of which shall fall within the protection scope of the present invention.

## Claims

1. A novel miniaturized chimeric antigen receptor, comprising an extracellular segment, a transmembrane region and an intracellular segment, wherein the extracellular segment comprises an antigen-binding region and a hinge region; the intracellular segment comprises a portion of CD3ζ in tandem with a co-stimulatory molecule; the chimeric antigen receptor has a length of equal to or less than 300 amino acids; the antigen-binding region of the extracellular segment has a length of equal to or less than 150 amino acids; the hinge region and the transmembrane region have a combined length of equal to or less than 70 amino acids; the intracellular segment has a length of equal to or less than 100 amino acids.

2. The chimeric antigen receptor according to claim 1, wherein the portion of CD3ζ is a CD3ζ ITAM domain.

3. The chimeric antigen receptor according to claim 2, wherein the CD3ζ ITAM domain comprises a CD3ζ ITAM1 domain, a CD3ζ ITAM2 domain or a CD3ζ ITAM3 domain.

4. The chimeric antigen receptor according to claim 1, wherein the antigen-binding region comprises an antibody targeting an antigen of a solid tumor or an antigen of a hematologic tumor cell.

5. The chimeric antigen receptor according to claim 1, wherein the antibody comprises a single-domain antibody or a nanobody.

6. The chimeric antigen receptor according to claim 1, wherein the co-stimulatory molecule is a single co-stimulatory molecule.

7. The chimeric antigen receptor according to claim 1, wherein the hinge region comprises a CD8 hinge region or a CD28 hinge region.

8. The chimeric antigen receptor according to claim 1, wherein the transmembrane region comprises a CD8 transmembrane region, a CD28 transmembrane region or a CD3 transmembrane region.

9. The chimeric antigen receptor according to claim 1, wherein the hinge region is connected to the transmembrane region, the connections comprising a CD8 hinge region connected to a CD8 transmembrane region, a CD28 hinge region connected to a CD28 transmembrane region, a CD28 hinge region connected to a CD3 transmembrane region, or a CD8 hinge region connected to a CD3 transmembrane region.

10. A CAR-T cell expressing the chimeric antigen receptor according to any one of claims 1 to 9.

11. Use of the CAR-T cell according to claim 10 in the manufacture of a medicament for the treatment of a solid tumor or a hematologic tumor.

12. Use of the CAR-T cell according to claim 10 in the manufacture of a medicament for the treatment of an autoimmune disease.

13. The use according to claim 11, wherein the solid tumor comprises one or more of ovarian cancer, breast cancer, gastric cancer, colorectal cancer, lymphoma and multiple myeloma; the hematologic tumor comprises leukemia.

14. The use according to claim 11 or claim 12, wherein the portion of CD3ζ of the CAR-T cell is used for achieving appropriate activation of downstream signalling of a T cell.
